(19) Europäisches Patentamt
European Patent Office
Office européen des brevets

(11) EP 2 978 036 B1

(12) **EUROPEAN PATENT SPECIFICATION**

(45) Date of publication and mention of the grant of the patent:
**04.03.2020 Bulletin 2020/10**

(21) Application number: **14767297.6**

(22) Date of filing: **20.03.2014**

(51) Int Cl.:
***H01L 51/30*** *(2006.01)* ***C07D 487/04*** *(2006.01)*
***C07D 493/04*** *(2006.01)* ***C07D 495/04*** *(2006.01)*
***C07D 513/04*** *(2006.01)* ***C07F 7/08*** *(2006.01)*
***C07F 7/10*** *(2006.01)* ***H01L 29/786*** *(2006.01)*

(86) International application number:
**PCT/JP2014/057827**

(87) International publication number:
**WO 2014/148614 (25.09.2014 Gazette 2014/39)**

(54) **ORGANIC THIN FILM TRANSISTOR**
ORGANISCHER DÜNNSCHICHTTRANSISTOR
TRANSISTOR À COUCHES MINCES ORGANIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2013 JP 2013059460**

(43) Date of publication of application:
**27.01.2016 Bulletin 2016/04**

(73) Proprietor: **FUJIFILM Corporation**
**Tokyo 106-8620 (JP)**

(72) Inventors:
- **TAKAKU, Koji**
**Ashigarakami-gun**
**Kanagawa 258-8577 (JP)**
- **HIRAI, Yuki**
**Ashigarakami-gun**
**Kanagawa 258-8577 (JP)**
- **SOTOYAMA, Wataru**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**WO-A1-2011/068950 WO-A1-2011/074232**
**JP-A- 2008 181 994 JP-A- 2009 218 333**
**JP-A- 2012 031 159 US-A1- 2011 253 944**

- **KWON JAE-HONG ET AL: "Organic thin film transistors using 6,13-bis(tri-isopropylsilylethynyl)pentace ne embedded into polymer binders", APPLIED PHYSICS LETTERS, AMERICAN INSTITUTE OF PHYSICS, 2 HUNTINGTON QUADRANGLE, MELVILLE, NY 11747, vol. 94, no. 1, 8 January 2009 (2009-01-08), pages 13506-13506, XP012118255, ISSN: 0003-6951, DOI: 10.1063/1.3063123**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 978 036 B1

## Description

### Technical Field

The present invention relates to an organic thin film transistor, an organic semiconductor thin film, and an organic semiconductor material. More specifically, the invention relates to a compound having a phenanthrene structure, an organic thin film transistor containing the compound, an organic semiconductor material for a non-light emitting organic semiconductor device containing the compound, a material for an organic thin film transistor containing the compound, a coating solution for a non-light emitting organic semiconductor device containing the compound, and an organic semiconductor thin film for a non-light emitting organic semiconductor device containing the compound.

### Background Art

A device using an organic semiconductor material is expected to have various advantages as compared to an ordinary device using an inorganic semiconductor material, such as silicon, and thus is receiving much attention. Examples of the device using an organic semiconductor material include a photoelectric conversion device, such as an organic thin film solar cell and a solid state image sensing device, using an organic semiconductor material as a photoelectric conversion material, and a non-light emitting organic transistor. The device using an organic semiconductor material has a possibility that a device having a large area may be produced at a low temperature and a low cost, as compared to a device using an inorganic semiconductor material. Furthermore, the characteristics of the material may be easily changed by changing the molecular structure thereof, and thus there is a wide range of variations in materials, by which functions and devices that have not been achieved by an inorganic semiconductor material may be realized.

For example, Patent Document 1 describes a compound having two phenanthrodipyrrole structures in the molecule, and describes that the compound may be used as an organic EL material, and describes that a high efficiency, a low-voltage, a high luminance and a long service-life are achieved.

Patent Document 2 describes an organic semiconductor compound having a polycyclic condensed ring structure with condensed pyrrole. However, Patent Document 2 does not describe or suggest the purpose of an organic transistor.

### Citation List

#### Patent Documents

[Patent Document 1] JP-A-2011-46687
[Patent Document 2] JP-A-2012-513459

### Summary of Invention

#### Technical Problem

It has been known that a polycyclic condensed compound containing an aromatic heterocyclic ring is useful as a material for an organic EL device, as described in Patent Documents 1 and 2. However, it may not be said that a compound that is useful as a material for an organic EL device is immediately useful as a semiconductor material for an organic thin film transistor. This is because there is a difference in the characteristics demanded for the organic compound between an organic EL device and an organic thin film transistor. An organic EL device generally requires charge transport in the thickness direction of the thin film (which is generally from several nanometers to several hundred nanometers), whereas an organic thin film transistor requires charge (carrier) transport in a long distance between electrodes in the plane direction of the thin film (which is generally from several micrometers to several hundred micrometers). Accordingly, the demanded carrier mobility is considerably high. Thus, as a semiconductor material for an organic thin film transistor, an organic compound that has a high alignment order of molecules with high crystallinity is demanded. Furthermore, for achieving a high carrier mobility, the $\pi$-conjugate plane is preferably perpendicular to the substrate. In an organic EL device, on the other hand, a device that has a high light emission efficiency and uniform in-plane light emission is demanded for enhancing the light emission efficiency. In general, an organic compound having high crystallinity may be a cause of light emission defects, such as in-plane electric field unevenness, in-plane light emission unevenness and light emission quenching, and thus the material for an organic EL device is demanded to have high amorphous property with low crystallinity. Accordingly, even when an organic compound constituting a material for an organic EL device is diverted to an organic semiconductor material, good transistor characteristics may not immediately

obtained.

Actually, Patent Document 1 describes the phenanthrodipyrrole compound, but phenanthrodipyrrole compound described in Patent Document 1 has failed to provide sufficient transistor characteristics (a low carrier mobility) since it cannot provide sufficient overlap of HOMO orbitals.

Patent Document 2 describes the organic semiconductor compound having a polycyclic condensed ring structure with condensed pyrrole, but Patent Document 2 does not describe an example of the application of the compound described therein to an organic transistor, and the use of the compounds described therein has failed to provide sufficient transistor characteristics (a low carrier mobility).

Under the circumstances, the inventors have made investigations for solving the problems in the related art. An object to be achieved by the invention is to provide an organic thin film transistor that has a high carrier mobility, a small change in the threshold voltage after repeated driving and a high solubility in an organic solvent.

Solution to Problem

As a result of earnest investigations for solving the problems, the inventors have found that an organic thin film that has advantageously a high carrier mobility, small change in the threshold voltage after repeated driving, and a high solubility in an organic solvent may be obtained by condensing thiophen, furan or pyrrole to provide sufficient overlap of HOMO orbitals in the phenanthene structure. Especially, the inventors have found that it shows small change in the threshold voltage after repeated driving, and thus have completed the invention.

The invention as a specific measure for solving the problems is defined in the claims.

Advantageous Effects of Invention

According to the invention, an organic thin film transistor may be provided that has a high carrier mobility, a small change in the threshold voltage after repeated driving and a high solubility in an organic solvent.

Brief Description of Drawings

[Fig. 1] Fig. 1 is a schematic illustration showing a cross sectional structure of one example of the organic thin film transistor of the invention.

[Fig. 2] Fig. 2 is a schematic illustration showing a cross sectional structure of an organic thin film transistor that is produced as a substrate for measuring FET characteristics in the example of the invention.

Description of Embodiments

The invention will be described in detail below. The description for the constitutional components shown below may be made with reference to representative embodiments and specific examples, but the invention is not limited to the embodiments and the examples. In the description, a numerical range expressed with reference to an upper limit and/or a lower limit means a range that includes the upper limit and/or the lower limit.

In the invention, the hydrogen atom that is referred without any particular discrimination in the description of the formulae herein includes isotopes thereof (such as a deuterium atom). The atoms constituting the substituents also include isotopes thereof.

[Organic Thin Film Transistor]

The organic thin film transistor of the invention contains a compound represented by the following formula (1) in a semiconductor active layer:

Formula (1)

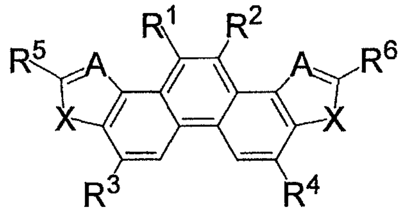

wherein in the formula (1), X represents an S atom, an O atom or $NR^7$; A each independently represents $CR^8$ or a nitrogen atom; and $R^1$ to $R^8$ each independently represent a hydrogen atom or a substituent according to the claims, provided that at least one of $R^1$ to $R^8$ represents a substituent.

According to the constitution, the organic thin film transistor of the invention has a high carrier mobility, a small change in the threshold voltage after repeated driving, and a high solubility in an organic solvent.

The compound represented by the formula (1) has an excellent HOMO orbital geometry and sufficient overlap of HOMO orbitals by condensing thiophen, furan or pyrrole such that hetero atoms are bonded to 2- and 7-position of the phenanthrene structure. Accordingly, an organic thin film transistor having a high carrier mobility may be obtained.

For reducing the change in the threshold voltage after repeated driving, there are such requirements as chemical stability of the organic semiconductor material (particularly, air oxidation resistance and redox stability), thermal stability in the form of a thin film, a large film density capable of preventing air and water from invading, a film quality with less defects capable of preventing charges from being accumulated, and the like. It is considered that the compound represented by the formula (1) satisfies these requirements and thus has a small change in the threshold voltage after repeated driving. Accordingly, the organic thin film transistor of the invention having a less change in the threshold voltage after repeated driving has a semiconductor active layer that has a high chemical stability, a high film density, and the like, and thus effectively functions as a transistor for a prolonged period of time.

Patent Document 1 describes the phenanthrodipyrrole compound which fails to have a preferable HOMO orbital geometry since the condensing position is different from that of the formula (1). Moreover, the substituent on the nitrogen atom has high bulkiness, and the compound has a low carrier mobility due to an insufficient overlap of HOMO orbitals. Patent Document 2 fails to describe a compound that has the same skeleton as the compound represented by the formula (1), but only recites a structural isomer of the compound represented by the formula (1). However, the structural isomer has a low carrier mobility.

In the invention, on the other hand, the advantageous effects of the invention may be obtained by using, the advantageous effects of the invention may be obtained by using, as an organic semiconductor material, the compound that has a skeleton represented by the formula (1) provides a sufficient overlap of HOMO orbitals, and unexpectedly a small change in the threshold voltage after repeated driving.

Preferred embodiments of the compound of the invention, the organic thin film transistor of the invention, and the like will be described below.

<Compound Represented by Formula (1)>

The compound of the invention is represented by the following formula (1). The compound of the invention is contained in a semiconductor active layer described later in the organic thin film transistor of the invention. Thus, the compound of the invention may be used as a material for an organic thin film transistor.

[Formula (1)]

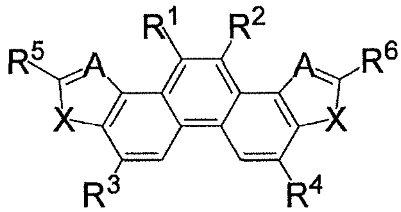

wherein in the formula (1), X represents an S atom, an O atom or $NR^7$; A each independently represents $CR^8$ or a nitrogen atom; and $R^1$ to $R^8$ each independently represent a hydrogen atom or a substituent according to the claims, provided that at least one of $R^1$ to $R^8$ represents a substituent.

In the formula (1), $R^1$ to $R^8$ each independently represent a hydrogen atom or a substituent according to the claims, provided that at least one of $R^1$ to $R^8$ represents a substituent.

Examples of the substituent that may be $R^1$ to $R^8$ independently in the formula (1) include a halogen atom, an alkyl group (including an alkyl group having from 1 to 11 carbon atoms such as a methyl group, an ethyl group, a propyl group, a butyl group, a pentyl group, a hexyl group, a heptyl group, an octyl group, a nonyl group, a decyl group, a undecyl group; a 2-ethylhexyl group; a cycloalkyl group; a bicycloalkyl group; and a tricycloalkyl group), an alkenyl group (including an 1-pentenyl group, a cycloalkenyl group and a bicycloalkenyl group), an alkynyl group (including an 1-pentynyl group, a trimethylsilylethynyl group, a triethylsilylethynyl group, a tri-i-propylsilylethynyl group, and a 2-p-propylphenylethynyl group), an aryl group (including an aryl group having from 6 to 20 carbon atoms such as a phenyl group, a naphthyl group, a p-pentylphenyl group, 3,4-dipentylphenyl group, a p-heptoxyphenyl group, and a 3,4-diheptoxyphenyl group), a heterocyclic group (including a 2-hexylfuranyl group), a cyano group, a hydroxyl group, a nitro group, an acyl group (including a hexanoyl group and a benzoyl group), an alkoxy group (including a butoxy group), an aryloxy group, a silyloxy group, a heterocyclic oxy group, an acyloxy group, a carbamoyloxy group, an amino group (including an anilino group), an acylamino group, an aminocarbonylamino group (including an ureido group), an alkoxy- or aryloxycarbonylamino group, an alkyl- or arylsulfonylamino group, a mercapto group, an alkyl- or arylthio group (including a methylthio group and an octylthio group), a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group, a hydrazino group, and other known groups.

These substituents may further include the substituents stated above. Furthermore, in the case where the compound represented by the formula (1) is a polymer compound with a repeated structure, $R^1$ to $R^8$ may have a group derived from a polymerizable group.

Among these, as examples of the substituent that may be $R^1$ to $R^6$ independently preferably include an alkyl group, an aryl group, an alkenyl group, an alkinyl group, a heterocyclic group, an alkoxy group, an alkylthio group, and a group represented by $-L^a-R^a$ stated below, and more preferably, an alkyl group having from 1 to 12 carbon atoms, an aryl group having from 6 to 20 carbon atoms, an alkenyl group having from 2 to 12 carbon atoms, an alkynyl group having from 2 to 12 carbon atoms, an alkoxy group having from 1 to 11 carbon atoms, a heterocyclic group having from 5 to 12 carbon atoms, an alkylthio group having from 1 to 12 carbon atoms and a group represented by $-L^a-R^a$ stated below.

In the formula (1), X represents an S atom, an O atom or $NR^7$, provided that $R^7$ represents a hydrogen atom or a substituent according to the claims.

X is preferably an S atom or an O atom. In the formula (1), two Xs are preferably the same.

$R^7$ is preferably a hydrogen atom, an alkyl group, an aryl group, and/or a group represented by $-L^d-R^d$ or $-L^e-R^e$, which are stated below, more preferably an alkyl group having from 1 to 14 carbon atom and/or a group represented by $-L^d-R^d$ or $-L^e-R^e$, and particularly preferably an alkyl group having from 4 to 12 carbon atom and/or a group represented by $-L^d-R^d$ or $-L^e-R^e$. $R^7$ is preferably a long-chain alkyl group, especially a long-chain, liner alkyl group from the standpoint of a high molecular linearity, which causes a high carrier mobility.

In the case where $R^7$ represents an alkyl group, it may be a liner alkyl group, a branched alkyl group or a cyclic alkyl group, but a linear alkyl group is preferred from the standpoint of a high molecular linearity, which causes a high carrier mobility.

$R^7$ is preferably a group represented by $-L^d-R^d$ or $-L^e-R^e$ from the standpoint of a high molecular linearity, which causes a high carrier mobility.

In the formula (1), A each independently represents $CR^8$ or a nitrogen atom. $CR^8$ is preferred. In the formula (1), two As may be the same or different, but it is preferable that they are the same.

$R^8$ is preferably a group having a linking chain length of 3.7 Å or less, more preferably length of from 1.0 to 3.7 Å, further preferably length of form 1.0 to 2.1 Å.

Herein, a linking chain length means a length from a C atom in a $C-R^8$ bond of the phenanthrene structure to the end of the substituent $R^8$. The structure optimization calculation may be performed by the density functional approach (Gaussian 03 (Gaussian, Inc., U.S.), base function: 6-31G*, exchange correlation function: B3LYP/LANL2DZ). The molecular lengths of representative substituents are 4.6 Å for a propyl group, 4.6 Å for a pyrrol group, 4.5 Å for a propynyl group, 4.6 Å for propenyl group, 4.5 Å for an ethoxy group, 3.7 Å for a methylthio group, 3.4 Å for an ethenyl group, 3.5 Å for an ethyl group, 3.6 Å for an ethynyl group, 3.3 Å for a methoxy group, 2.1 Å for a methyl group, and 1.0 Å for a hydrogen atom.

$R^8$ is preferably a hydrogen atom, a substituted or unsubstituted alkyl group having 2 or less carbon atoms, a substituted or unsubstituted alkinyl group having 2 or less carbon atoms, a substituted or unsubstituted alkenyl group having 2 or less carbon atoms or a substituted or unsubstituted acyl group having 2 or less carbon atoms, more preferably a hydrogen atom, or a substituted or unsubstituted alkyl group having 2 or less carbon atoms, and particularly preferably a hydrogen atom.

In the case where $R^8$ represents a substituted or unsubstituted alkyl group having 2 or less carbon atoms,

examples of the substituent that the alkyl group may choose include a cyano group, a fluorine group and a deuterium atom, but a cyano group is preferred. A substituted or unsubstituted alkyl group having 2 or less carbon atoms, which is represented by $R^8$, is preferably a methyl group, an ethyl group or a methyl group substituted with a cyano group, more preferably a methyl group or a methyl group substituted with a cyano group, and particularly preferably a methyl group substituted with a cyano group.

In the case where $R^8$ represents a substituted or unsubstituted alkynyl group having 2 or less carbon atoms, examples of the substituent that the alkynyl group may choose include a deuterium atom. A substituted or unsubstituted alkynyl group having 2 or less carbon atoms, which is represented by $R^8$, is an ethynyl group or an acetylene group substituted by a deuterium atom, preferably ethynyl group.

In the case where $R^8$ represents a substituted or unsubstituted alkenyl group having 2 or less carbon atoms, examples of the substituent that the alkenyl group may choose include a deuterium atom. A substituted or unsubstituted alkynyl group having 2 or less carbon atoms, which is represented by $R^8$, is an ethenyl group or an ethenyl group substituted by a deuterium atom, preferably ethenyl group.

In the case where $R^8$ represents a substituted or unsubstituted acyl group having 2 or less carbon atoms, examples of the substituent that the acyl group may choose include a fluorine atom. A substituted or unsubstituted acyl group having 2 or less carbon atoms, which is represented by $R^8$, is a formyl group, is an acetyl group, or an acetyl group substituted with a fluorine atom, preferably a formyl group.

In the present invention, the compound represented by the formula (1) is preferably a compound represented the formula (2-1), (2-2) or (2-3), more preferably a compound represented by the formula (2-1) or (2-2), and particularly preferably a compound represented by the formula (2-1) from the standpoint of the high solubility while a compound represented by the formula (2-2) from the standpoint of the high mobility.

A case in which a compound represented by the formula (1) is a compound represented by the formula (2-1) will be described below.

Formula (2-1)

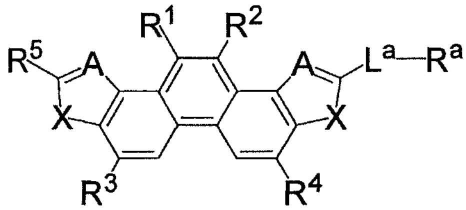

wherein in the formula (2-1), X represents an S atom, an O atom or $NR^7$; A each independently represents $CR^8$ or a nitrogen atom; $R^1$ to $R^5$, $R^7$ and $R^8$ each independently represent a hydrogen atom or a substituent according to the claims, provided that $R^5$ is not represented by $-L^a-R^a$; $L^a$ each independently represents a divalent linking group represented by any one of the following formulae (L-1) to (L-12), or a divalent linking group consisting of 2 or more divalent linking groups each represented by any one of the following formulae (L-1) to (L-12) in which the 2 or more divalent linking groups are bonded sequentially; and $R^a$ each independently represents a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^a$ represents a substituted or unsubstituted trialkylsilyl group only when $L^a$ bonded to $R^a$ is a divalent linking group represented by the following formula (L-3), and $R^a$ represents a hydrogen atom only when $L^a$ bonded to $R^a$ is a divalent linking group represented by any one of the following formulae (L-1) to (L-3) and (L-10) to (L-12).

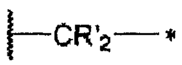

(L-1)

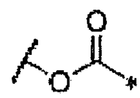

(L-8)

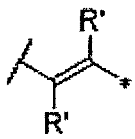

(L-2)

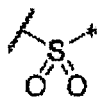

(L-9)

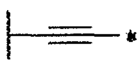

(L-3)

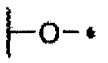 (L-4) 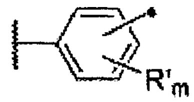 (L-10)

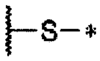 (L-5) 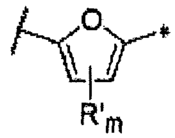 (L-11)

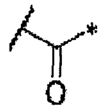 (L-6)

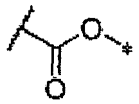 (L-7) 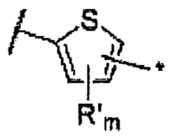 (L-12)

wherein in the formulae (L-1) to (L-12), the wavy line represents a position bonded to the phenanthrodifuran, phenanthrodithiphen or phenanthrodipyrrole skeleton, and * represents a position bonded to $R^a$; in the formula (L-10), m represents 4; in the formulae (L-11) and (L-12), m represents 2; in the formulae (L-1), (L-2), (L-10), (L-11) and (L-12), R' each independently represents a hydrogen atom or a substituent; and in the formulae (L-1) and (L-2), R' each independently may form a condensed ring with $R^a$ bonded to $L^a$.

In the formula (2-1), X represents an S atom, an O atom or $NR^7$, and $R^7$ represents a hydrogen atom or a substituent. A represents $CR^8$ or a nitrogen atom. X, $R^7$, $R^8$ and A in the formula (2-1) are the same as X, $R^7$, $R^8$ and A in the formula (1), respectively. The preferred ranges of them are the same as the preferred ranges of those in the formula (1).

Moreover, in the formula (2-1), $R^1$ to $R^5$ each independently represent a hydrogen atom or a substituent according to claims. $R^1$ to $R^5$ in the formula (2-1) are the same as $R^1$ to $R^6$ in the formula (1), respectively. The preferred ranges of them are the same as the preferred ranges of those in the formula (1).

In the formula (2-1), $L^a$ represents a divalent linking group consisting of divalent linking group (s) each represented by any one of the following formulae (L-1) to (L-12) in which the divalent linking groups are bonded sequentially.

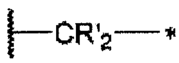 (L-1) 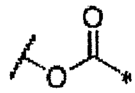 (L-8)

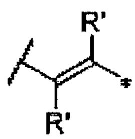 (L-2) 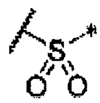 (L-9)

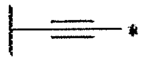 (L-3)

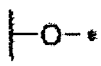 (L-4) 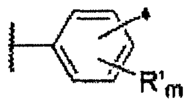 (L-10)

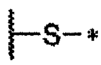 (L-5) 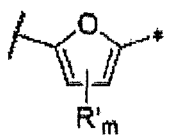 (L-11)

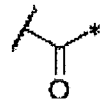 (L-6)

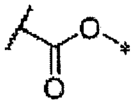 (L-7) 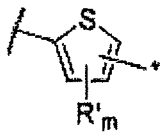 (L-12)

In the formulae (L-1) to (L-12), the wavy line represents a position bonded to the phenanthrodifuran, phenanthrodithiphen or phenanthrodipyrrole skeleton, and * represents a position bonded to $R^a$; in the formula (L-10), m represents 4; in the formulae (L-11) and (L-12), m represents 2; in the formulae (L-1), (L-2), (L-10), (L-11) and (L-12), R' each independently represents a hydrogen atom or a substituent; and in the formulae (L-1) and (L-2), R' each independently may form a condensed ring with $R^a$ bonded to $L^a$.

In the case where $L^a$ represents a divalent linking group consisting of divalent linking groups each represented by any one of the formulae (L-1) to (L-12) in which the divalent linking groups are bonded sequentially, the number of the divalent linking groups each represented by any one of the formulae (L-1) to (L-12) bonded sequentially is preferably from 2 to 4, and more preferably 2 or 3.

Particularly in the formulae (L-10) to (L-12), it is also preferred that any one of the formulae (L-1) to (L-12) is further inserted between * and $R^a$ to form $L^a$ that represents a linking group consisting of divalent linking groups each represented by any one of the formulae (L-1) to (L-12) in which the divalent linking groups are bonded sequentially.

Examples of the substituent R' in the formulae (L-1), (L-2), (L-10), (L-11) and (L-12) include the groups that are shown as examples of the other substituent that may be $R^1$ to $R^8$ in the formula (1).

In the formula (L-10), m represents 4; and in the formulae (L-11) and (L-12), m represents 2.

A divalent linking group represented by the formula (L-10) is a divalent linking group represented by any one of the following formulae (L-10A), (L-10B) and (L-10C), preferably a divalent linking group represented by any one of the following formulae (L-10A) and (L-10B), more preferably a divalent linking group represented by the following formula (L-10B).

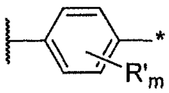 (L-10A) 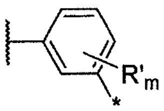 (L-10B) 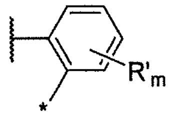 (L-10C)

A divalent linking group represented by the formula (L-12) is a divalent linking group represented by any one of the following formulae (L-12A), (L-12B) and (L-12C), preferably a divalent linking group represented by any one of the following formulae (L-12A) and (L-12B), more preferably a divalent linking group represented by the following formula (L-12B).

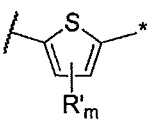 (L-12A) 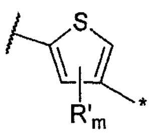 (L-12B) 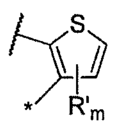 (L-12C)

L preferably represents a divalent linking group represented by any one of the formulae (L-1) to (L-6), (L-10), (L-11) and (L-12) or a divalent linking group consisting of 2 or more of the divalent linking groups in which 2 or more of the divalent linking groups are bonded sequentially, more preferably a divalent linking group represented by any one of the formulae (L-1) to (L-5), (L-10), (L-11) and (L-12) or a divalent linking group consisting of 2 or more of the divalent linking groups in which 2 or more of the divalent linking groups are bonded sequentially, further preferably a divalent linking group represented by any one of the formulae (L-1), (L-3), (L-10), (L-11) and (L-12) or a divalent linking group consisting of 2 or more of the divalent linking groups in which 2 or more of the divalent linking groups are bonded sequentially, from the standpoint of the chemical stability and the carrier transport property, particularly preferably a divalent linking group represented by any one of the formulae (L-1), (L-3), (L-10) and (L-12) or a divalent linking group consisting of 2 or more of the divalent linking groups in which 2 or more of the divalent linking groups are bonded sequentially, and further particularly preferably a divalent linking group represented by the formula (L-1) or (L-3) or a divalent linking group consisting of 2 or more of the divalent linking groups in which 2 or more of the divalent linking groups are bonded sequentially.

In the formula (2-1), $R^a$ represents a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^a$ represents a substituted or unsubstituted trialkylsilyl group only when $L^a$ bonded to $R^a$ is a divalent linking group represented by the following formula (L-3), and $R^a$ represents a hydrogen atom only when $L^a$ bonded to $R^a$ is a divalent linking group represented by any one of the following formulae (L-1) to (L-3) and (L-10) to (L-12).

In the case where $R^a$ represents a substituted or unsubstituted alkyl group having 1 or more carbon atoms, the number of carbon atoms thereof is preferably from 2 to 18, more preferably from 3 to 12 from the standpoint of the chemical stability and the carrier transport property, further preferably from 4 to 10, particularly preferably from 4 to 8, and further particularly preferably from 4 to 6.

In the compound represented by the formula (1), in the case where $L^a$-$R^a$ contains an alkyl group, a high carrier mobility may be obtained when the alkyl group represented by $R^a$ has a carbon number that is the lower limit of the aforementioned range or more. In the case where $L^a$ contains the formula (L-1) bonded to $R^a$, a high carrier mobility may be obtained when the alkyl group formed by bonding the alkylene group represented by the formula (L-1) and the alkyl group represented by $R^a$ has a carbon number that is the lower limit of the aforementioned range or more.

The alkyl group that may be $R^a$ may be any one of linear, branched and cyclic, and is preferably a linear alkyl group from the standpoint of the enhancement of the carrier mobility, more preferably a linear alkyl group having from 1 to 12 carbon atoms, further preferably a linear alkyl group having from 3 to 12 carbon atoms, particularly preferably a linear alkyl group having from 4 to 10 carbon atoms, further particularly preferably a linear alkyl group having from 4 to 8 carbon atoms, and still further particularly preferably a linear alkyl group having from 4 to 6 carbon atoms. In the case where R represents an alkyl group having a substituent, examples of the substituent include a halogen atom, and a fluorine atom is preferred. In the case where R represents an alkyl group having a fluorine atom, the alkyl group may be a perfluoroalkyl group, in which all the hydrogen atoms of the alkyl group are replaced by fluorine atoms.

In the case where $R^a$ represents an oligooxyethylene group having a repeating number of an oxyethylene group of 2 or more, the oxyethylene group represented by $R^a$ herein means a group represented by $-(OCH_2CH_2)_xOY$ (wherein the repeating number of an oxyethylene unit x is an integer of 2 or more, and Y as the terminal group represents a hydrogen atom or a substituent). In the case where Y as the terminal group of the oligooxyethylene group is a hydrogen atom, the group is a hydroxyl group. The repeating number of an oxyethylene unit x is preferably from 2 to 4, and more preferably from 2 to 3. The terminal hydroxyl group of the oligooxyethylene group is preferably blocked, i.e., Y preferably represents a substituent. In this case, the hydroxyl group is preferably blocked with an alkyl group having from 1 to 3 carbon atoms, i.e., Y preferably represents an alkyl group having from 1 to 3 carbon atoms, and Y more preferably represents a methyl group or an ethyl group, and particularly preferably a methyl group.

In the case where $R^a$ represents an oligosiloxane group having 2 or more silicon atoms, the repeating number of a siloxane unit is preferably from 2 to 4, and more preferably from 2 to 3. The Si atom is preferably bonded to a hydrogen atom or an alkyl group. In the case where the Si atom is bonded to an alkyl group, the number of carbon atoms of the alkyl group is preferably from 1 to 3, and for example, a methyl group or an ethyl group is preferably bonded thereto. The Si atom may be bonded to the same alkyl groups or may be bonded to different alkyl groups or a hydrogen atom. The siloxane units constituting the oligosiloxane group may be all the same as each other or different from each other, and are preferably all the same as each other.

$R^a$ is a substituted or unsubstituted trialkylsilyl group only when $L^a$ bonded to $R^a$ is a divalent linking group represented by the formula (L-3). In the case where $R^a$ is a substituted or unsubstituted trialkylsilyl group, the number of carbon atoms of the alkyl group, which is bonded to an Si atom, is preferably from 1 to 3. For example, a methyl group, an ethyl group or an isopropyl group is preferably bended. The same or different alkyl groups may be bonded to the Si atom. In the case where the $R^a$ is a trialkylsilyl group having substituent(s), the substituents are not especially limited.

A case in which a compound represented by the formula (1) is a compound represented by the formula (2-2) will be described below.

Formula (2-2)

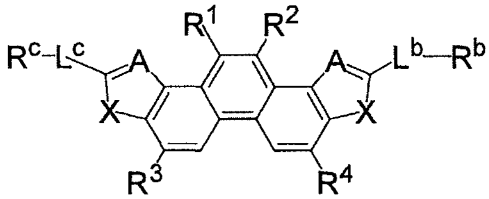

wherein in the formula (2-2), X represents an S atom, an O atom or $NR^7$; A represents $CR^8$ or a nitrogen atom; $R^1$ to $R^4$, $R^7$ and $R^8$ each independently represent a hydrogen atom or a substituent according to the claims; $L^b$ and $L^c$ each independently represent a divalent linking group represented by any one of the following formulae (L-1) to (L-12), or a divalent linking group consisting of 2 or more divalent linking groups each represented by any one of the following formulae (L-1) to (L-12) in which the 2 or more divalent linking groups are bonded sequentially; and $R^b$ and $R^c$ each

independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^b$ and $R^c$ represent a substituted or unsubstituted trialkylsilyl group only when $L^b$ or $L^c$ bonded to $R^b$ or $R^c$ is a divalent linking group represented by the following formula (L-3), and $R^b$ and $R^c$ represent a hydrogen atom only when $L^b$ or $L^c$ bonded to $R^b$ or $R^c$ is a divalent linking group represented by any one of the following formulae (L-1) to (L-3) and (L-10) to (L-12).

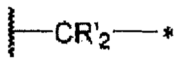 (L-1)

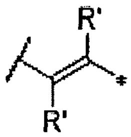 (L-2)

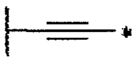 (L-3)

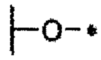 (L-4)

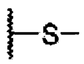 (L-5)

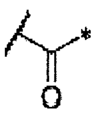 (L-6)

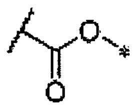 (L-7)

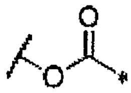 (L-8)

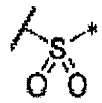 (L-9)

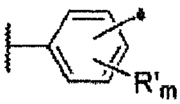 (L-10)

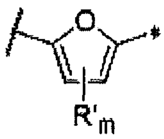 (L-11)

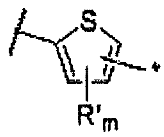 (L-12)

wherein in the formulae (L-1) to (L-12), the wavy line represents a position bonded to the phenanthrodifuran, phenanthrodithiphen or phenanthrodipyrrole skeleton, and * represents a position bonded to any one of $R^b$ to $R^c$; in the formula (L-10), m represents 4; in the formulae (L-11) and (L-12), m represents 2; in the formulae (L-1), (L-2), (L-10), (L-11) and (L-12), R' each independently represents a hydrogen atom or a substituent; and in the formulae (L-1) and (L-2), R' each independently may form a condensed ring with $R^b$ or $R^c$ each bonded to $L^b$ to $L^c$.

In the formula (2-2), X represents an S atom, an O atom or $NR^7$, and $R^7$ represents a hydrogen atom or a substituent according to the claims. A represents $CR^8$ or a nitrogen atom. X, $R^7$, $R^8$ and A in the formula (2-2) are the same as X, $R^7$, $R^8$ and A in the formula (1), respectively. The preferred ranges of them are the same as the preferred ranges of those in the formula (1).

Moreover, in the formula (2-2), $R^1$ to $R^4$ each independently represent a hydrogen atom or a substituent. $R^1$ to $R^4$ in the formula (2-2) are the same as $R^1$ to $R^6$ in the formula (1), respectively. The preferred ranges of them are the same as the preferred ranges of those in the formula (1).

In the formula (2-2), $L^b$ and $L^c$ each independently represent a divalent linking group represented by any one of the formulae (L-1) to (L-12), or a divalent linking group consisting of 2 or more divalent linking groups each represented by any one of the formulae (L-1) to (L-12) in which the 2 or more divalent linking groups are bonded sequentially. The preferred ranges of $L^b$ and $L^c$ are the same as the preferred ranges of $L^a$ in the formula (2-1) . It is preferable that $R^b$ and $R^c$ are the same.

In the formula (2-2), $R^b$ and $R^c$ each independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group. The preferred ranges of $R^b$ and $R^c$ are the same as the preferred ranges of $R^a$ in the formula (2-1). It is preferable that $R^b$ and $R^c$ are the same.

A case in which a compound represented by the formula (1) is a compound represented by the formula (2-3) will be described below.

Formula (2-3)

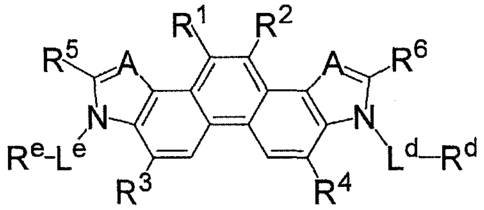

wherein in the formula (2-3), A represents $CR^8$ or a nitrogen atom; $R^1$ to $R^6$, and $R^8$ each independently represent a hydrogen atom or a substituent; $L^d$ and $L^e$ each independently represent a divalent linking group represented by any one of the following formulae (L-1) to (L-3), (L-6), (L-7), and (L-9) to (L-12), or a divalent linking group consisting of 2 or more divalent linking groups each represented by any one of the following formulae (L-1) to (L-12) in which the 2 or more divalent linking groups are bonded sequentially; and $R^d$ and $R^e$ each independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^d$ and $R^e$ represent a substituted or unsubstituted trialkylsilyl group only when $L^d$ or $L^e$ bonded to $R^d$ or $R^e$ is a divalent linking group represented by the following formula (L-3), and $R^d$ and $R^e$ represent a hydrogen atom only when $L^d$ or $L^e$ bonded to $R^d$ or $R^e$ is a divalent linking group represented by any one of the following formulae (L-1) to (L-3) and (L-10) to (L-12).

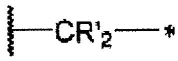 (L-1)

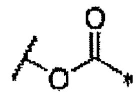 (L-8)

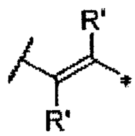 (L-2)

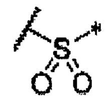 (L-9)

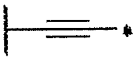 (L-3)

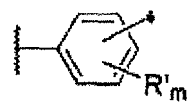 (L-10)

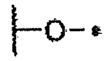 (L-4)

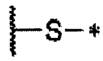 (L-5)

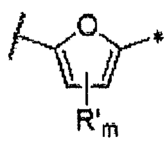 (L-11)

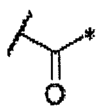 (L-6)

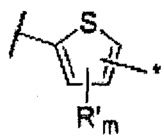 (L-12)

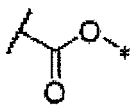 (L-7)

wherein in the formulae (L-1) to (L-12), the wavy line represents a position bonded to the phenanthrodifuran, phenanthrodithiphen or phenanthrodipyrrole skeleton, and * represents a position bonded to any one of $R^d$ to $R^e$; in the formula (L-10), m represents 4; in the formulae (L-11) and (L-12), m represents 2; in the formulae (L-1), (L-2), (L-10), (L-11) and (L-12), R' each independently represents a hydrogen atom or a substituent; and in the formulae (L-1) and (L-2), R' each independently may form a condensed ring with $R^d$ or $R^e$ each bonded to $L^d$ to $L^e$.

In the formula (2-3), A represents $CR^8$ or a nitrogen atom. A and $R^8$ in the formula (2-3) are the same as A and $R^8$ in the formula (1), respectively. The preferred ranges of them are the same as the preferred ranges of those in the formula (1) .

In the formula (2-3), $R^1$ to $R^6$ each independently represent a hydrogen atom or a substituent according to the

claims. $R^1$ to $R^6$ in the formula (2-3) are the same as $R^1$ to $R^6$ in the formula (1), respectively. The preferred ranges of them are the same as the preferred ranges of those in the formula (1).

In the formula (2-3), $L^d$ and $L^e$ each independently represent a divalent linking group represented by any one of the following formulae (L-1) to (L-12), or a divalent linking group consisting of 2 or more divalent linking groups each represented by any one of the following formulae (L-1) to (L-12) in which the 2 or more divalent linking groups are bonded sequentially.

In the case where $L^d$ and $L^e$ represent a divalent linking group consisting of divalent linking groups each represented by any one of the formulae (L-1) to (L-12) in which divalent linking groups are bonded sequentially, the number of the divalent linking groups each represented by any one of the formulae (L-1) to (L-12) in which the divalent linking groups are bonded sequentially is preferably from 2 to 4, and more preferably 2 or 3.

Particularly in the formulae (L-10) to (L-12), it is also preferred that any one of the formulae (L-1) to (L-12) is further inserted between * and $R^d$ or between * and $R^e$ to form $L^d$ and $L^e$ that represent linking groups consisting of divalent linking groups each represented by any one of the formulae (L-1) to (L-12) in which the divalent linking groups are bonded sequentially.

Examples of the substituent R' in the formulae (L-1), (L-2), (L-10), (L-11) and (L-12) include the groups that are shown as examples of the other substituent that may be $R^1$ to $R^8$ in the formula (1).

In the formula (L-10), m represents 4; and in the formulae (L-11) and (L-12), m represents 2.

$L^d$ and $L^e$ each preferably represent a divalent linking group represented by any one of the formulae (L-1) to (L-6), (L-10), (L-11) and (L-12) or a divalent linking group consisting of 2 or more of the divalent linking groups bonded sequentially, more preferably a divalent linking group represented by any one of the formulae (L-1) to (L-5), (L-10), (L-11) and (L-12) or a divalent linking group consisting of 2 or more of the divalent linking groups bonded sequentially, further preferably a divalent linking group represented by any one of the formulae (L-1), (L-3), (L-10), (L-11) and (L-12) or a divalent linking group consisting of 2 or more of the divalent linking groups bonded sequentially, from the standpoint of the chemical stability and the carrier transport property, particularly preferably a divalent linking group represented by any one of the formulae (L-10), (L-11) and (L-12) or a divalent linking group consisting of 2 or more of the divalent linking groups bonded sequentially, and further particularly preferably a divalent linking group represented by the formula (L-10), (L-11) or (L-12). $L^d$ and $L^e$ are preferably the same as each other.

In the formula (2-3), $R^d$ and $R^e$ each independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group. The preferred ranges of $R^d$ and $R^e$ are the same as the preferred ranges of $R^a$ in the formula (2-1). $R^d$ and $R^e$ are preferably the same as each other.

In the formulae (2-1), (2-2) and (2-3), all of $R^a$ to $R^e$ each preferably represent a substituted or unsubstituted alkyl group, more preferably a substituted or unsubstituted alkyl group having 2 or more carbon atoms, particularly preferably a substituted or unsubstituted linear alkyl group having from 3 to 12 carbon atoms, and further particularly preferably a substituted or unsubstituted linear alkyl group having from 4 to 10 carbon atoms. In the formulae (2-1), (2-2) or (2-3), all of $R^a$ to $R^e$ each preferably represent a unsubstituted alkyl group, among substituted or unsubstituted alkyl groups.

In the formulae (2-1), (2-2) and (2-3), all of $L^a$ to $L^e$ each preferably represent a divalent linking group represented by any one of the formulae (L-1) to (L-5), (L-10), (L-11) and (L-12) or a divalent linking group consisting of 2 or more of the divalent linking groups bonded seqentially.

In the formulae (2-1) and (2-2), all of $L^a$ to $L^e$ each more preferably represent a divalent linking group represented by any one of the formulae (L-1) to (L-3), (L-10), and (L-12) or a divalent linking group consisting of 2 or more of the divalent linking groups bonded sequentially, and particularly preferably a divalent linking group represented by any one of the formulae (L-1) to (L-3), (L-10), and (L-12), from the standpoint of the chemical stability and the carrier transport property.

In the formula (2-3), $L^d$ and $L^e$ each more preferably represent a divalent linking group represented by any one of the formulae (L-10), (L-11) and (L-12) or a divalent linking group consisting of 2 or more of the divalent linking groups bonded sequentially, and particularly preferably a divalent linking group represented by any one of the formulae (L-10), (L-11) and (L-12), from the standpoint of the chemical stability and the carrier transport property.

Specific examples of the compound represented by the formula (1) are shown below, but the compound represented by the formula (1) capable of being used in the invention is not construed as being limited to the specific examples.

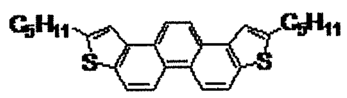

Compound 1

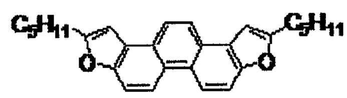

Compound 2

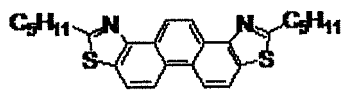

Compound 3

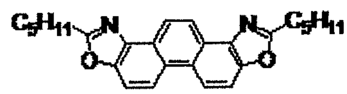

Compound 4

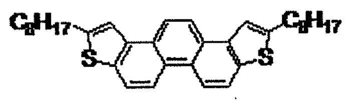

Compound 5

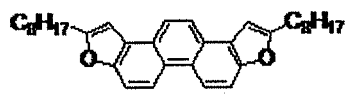

Compound 6

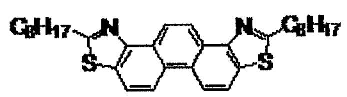

Compound 7

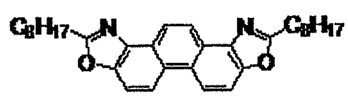

Compound 8

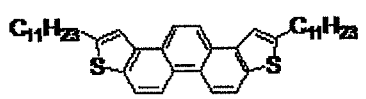

Compound 9

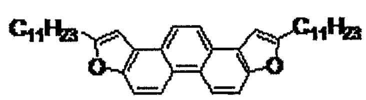

Compound 10

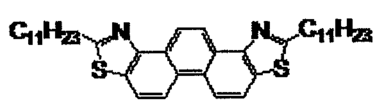

Compound 11

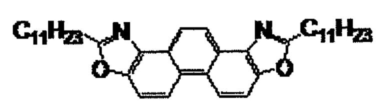

Compound 12

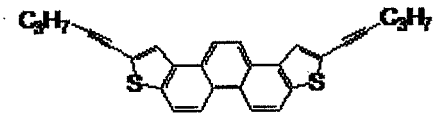

Compound 13

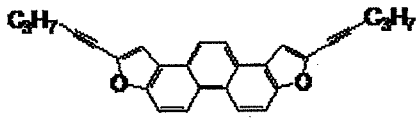

Compound 14

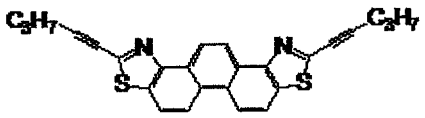

Compound 15

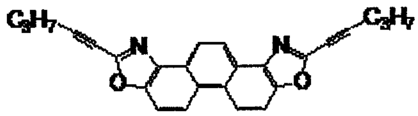

Compound 16

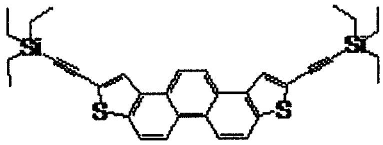

Compound 17

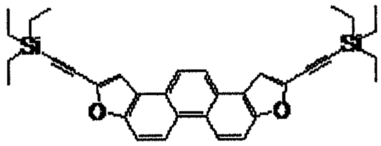

Compound 18

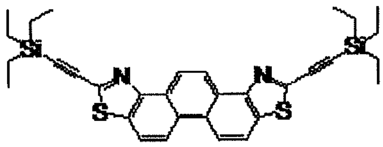

Compound 19

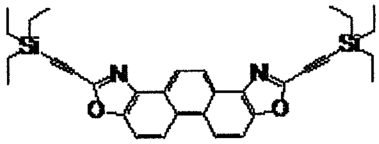

Compound 20

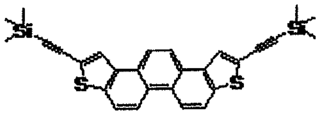

Compound 21

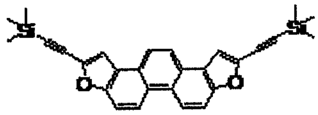

Compound 22

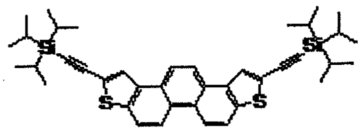

Compound 23

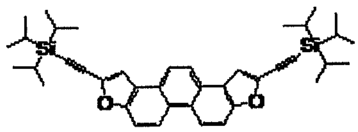

Compound 24

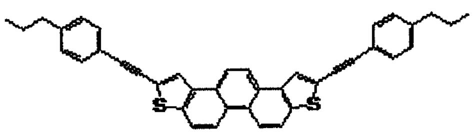

Compound 25

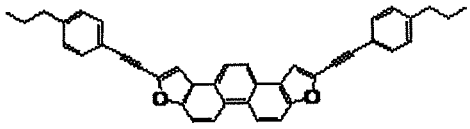

Compound 26

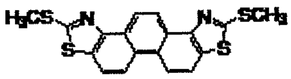

Compound 27

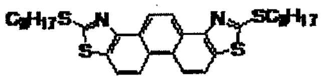

Compound 28

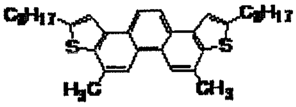

Compound 29

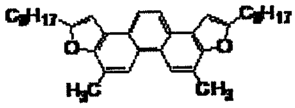

Compound 30

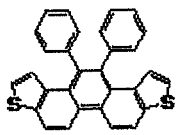

Compound 31

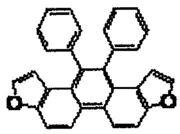

Compound 32

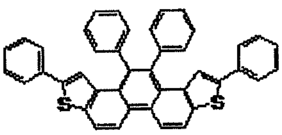

Compound 33

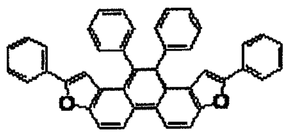

Compound 34

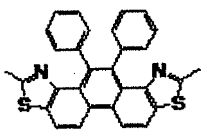

Compound 35

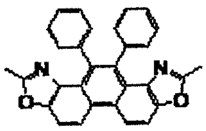

Compound 36

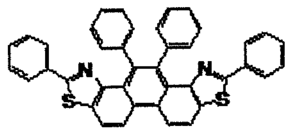

Compound 37

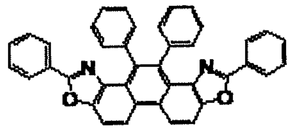

Compound 38

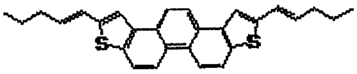

Compound 39

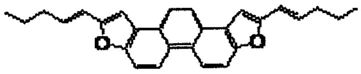

Compound 40

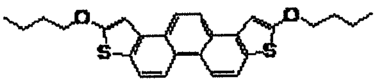

Compound 41

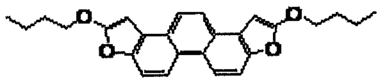

Compound 42

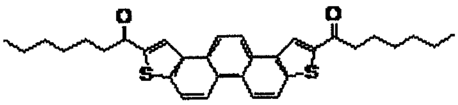

Compound 43

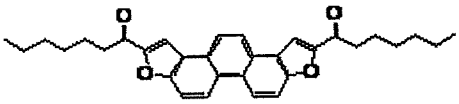

Compound 44

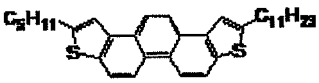

Compound 45

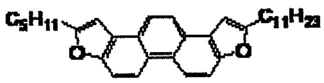

Compound 46:

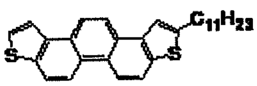

Compound 47

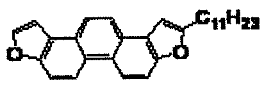

Compound 48

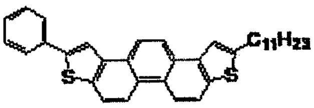

Compound 49

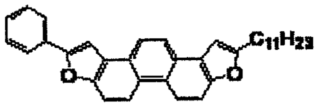

Compound 50

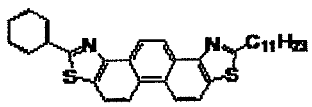

Compound 51

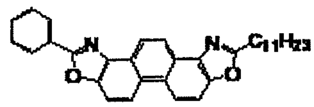

Compound 52

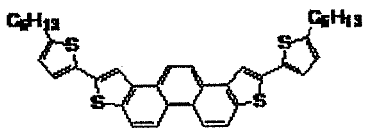

Compound 53

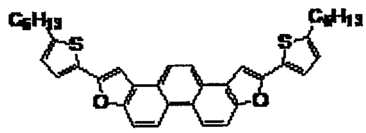

Compound 54

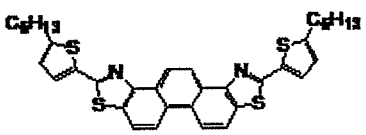

Compound 55

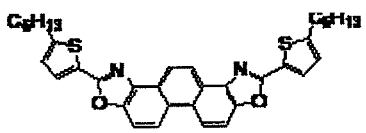

Compound 56

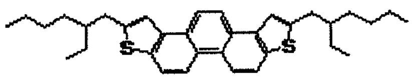

Compound 57

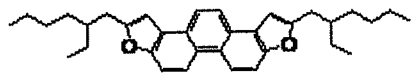

Compound 58

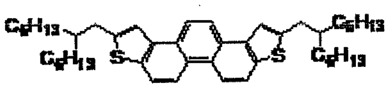

Compound 59

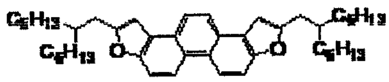

Compound 60

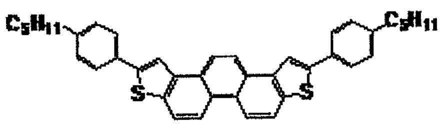

Compound 61

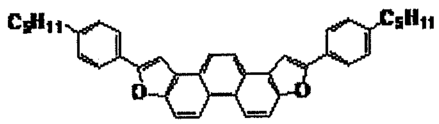

Compound 62

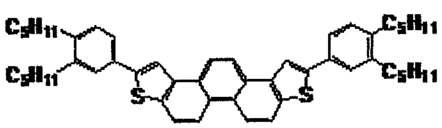

Compound 63

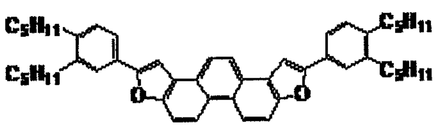

Compound 64

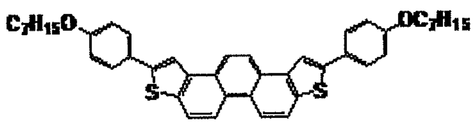

Compound 65

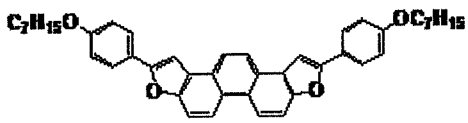

Compound 66

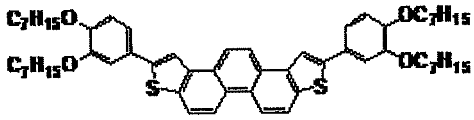

Compound 67

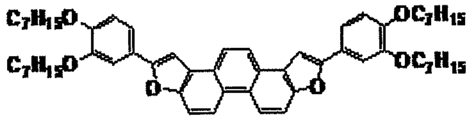

Compound 68

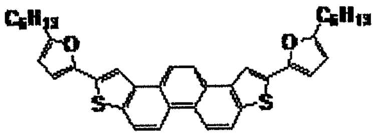

Compound 69

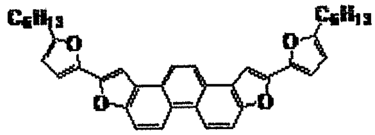

Compound 70

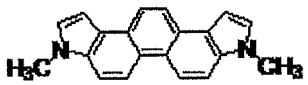

Compound 71

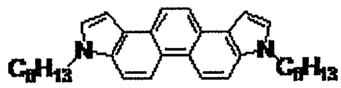

Compound 72

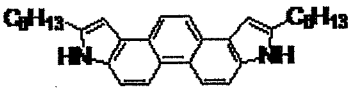

Compound 73

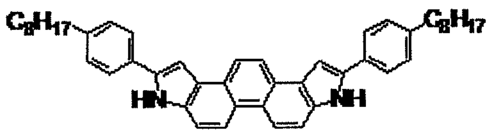

Compound 74

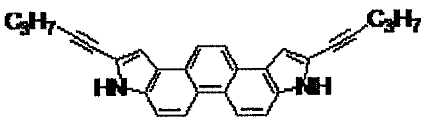

Compound 75

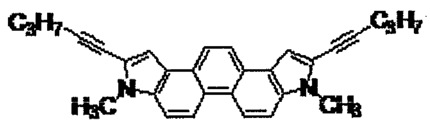

Compound 76

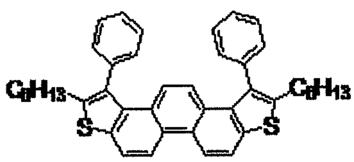

Compound 77

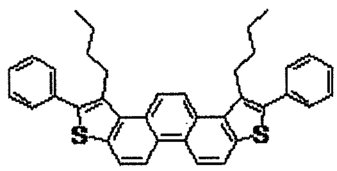

Compound 78

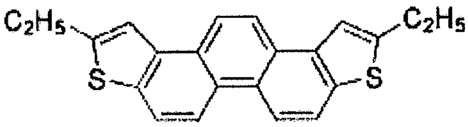

Compound 79

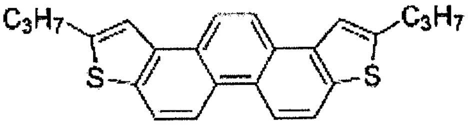

Compound 80

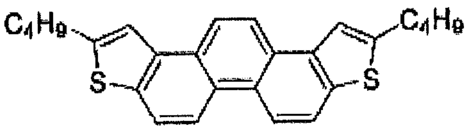

Compound 81

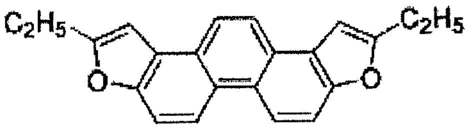

Compound 82

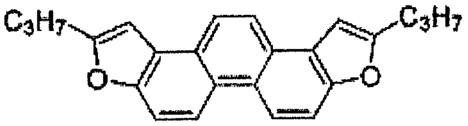

Compound 83

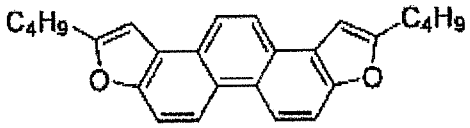

Compound 84

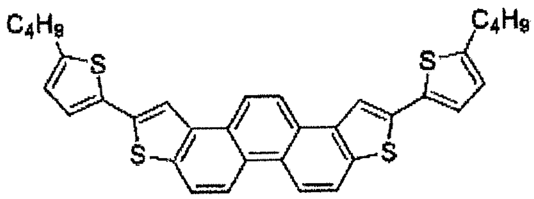

Compound 85

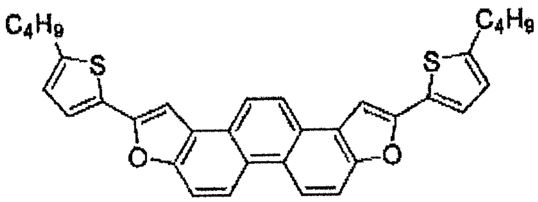

Compound 86

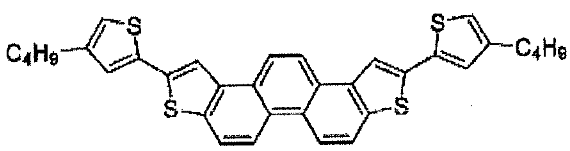

Compound 87

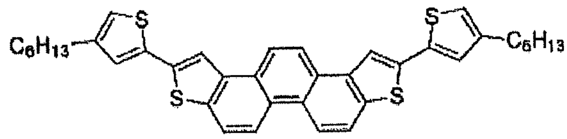

Compound 88

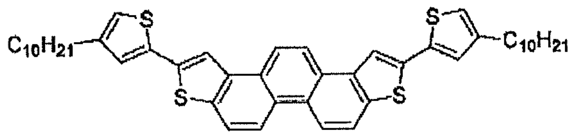

Compound 89

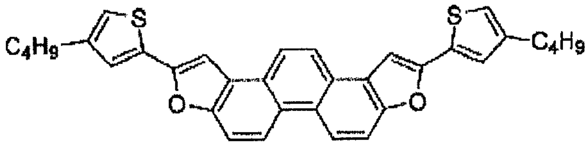

Compound 90

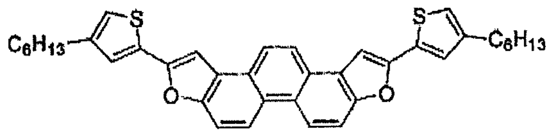

Compound 91

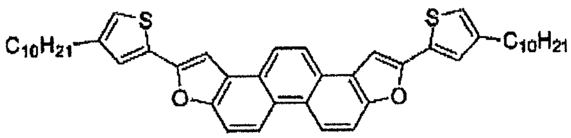

Compound 92

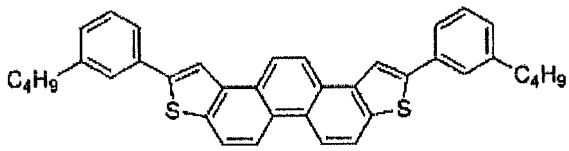

Compound 93

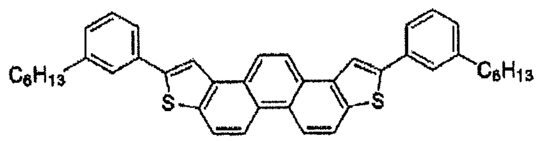

Compound 94

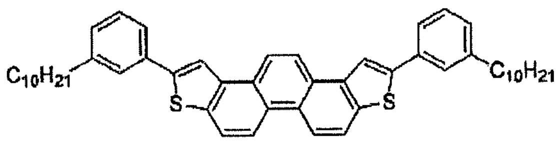

Compound 95

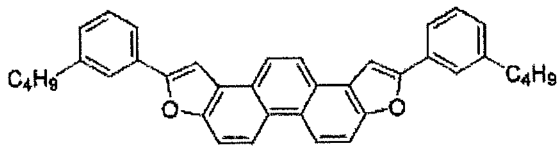

Compound 96

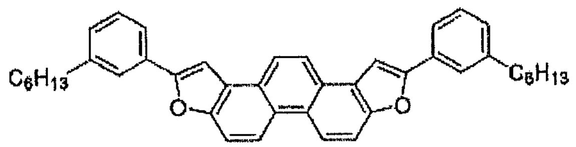

Compound 97

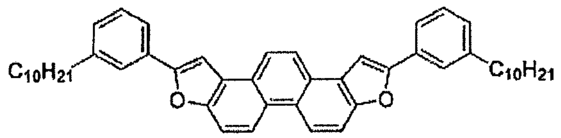

Compound 98

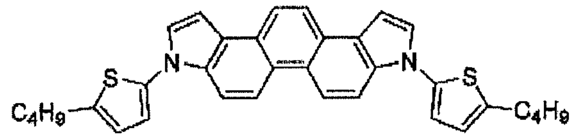

Compound 99

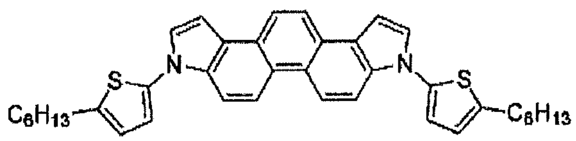

Compound 100

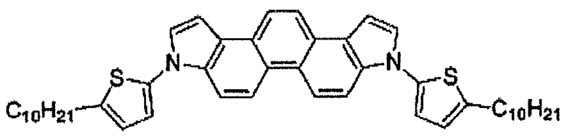

Compound 101

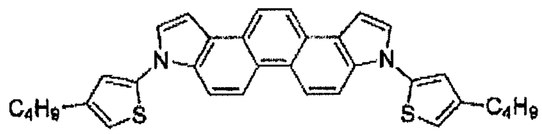

Compound 102

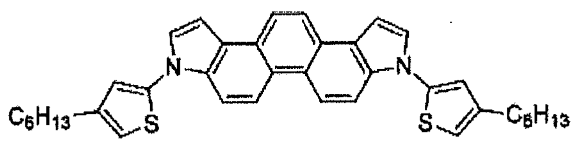

Compound 103

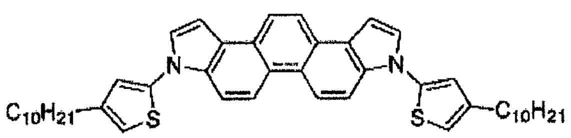

Compound 104

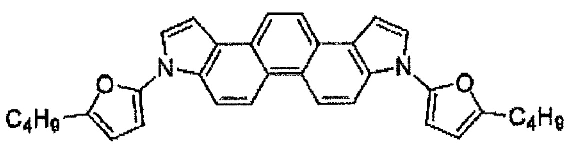

Compound 105

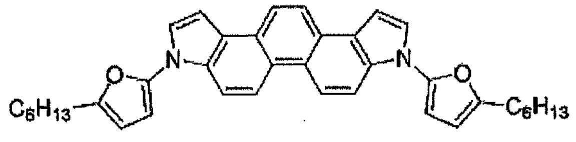

Compound 106

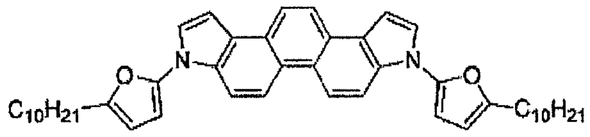

Compound 107

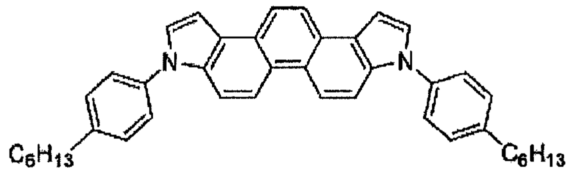

Compound 108

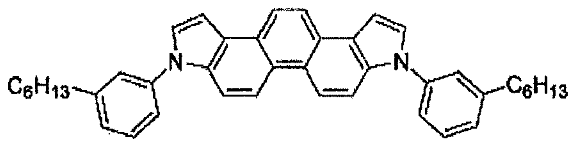

Compound 109

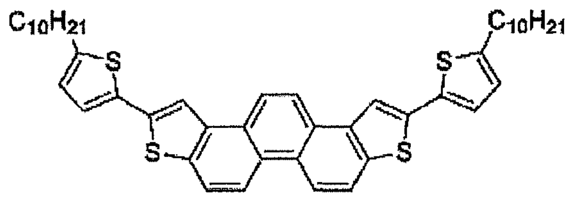

Compound 110

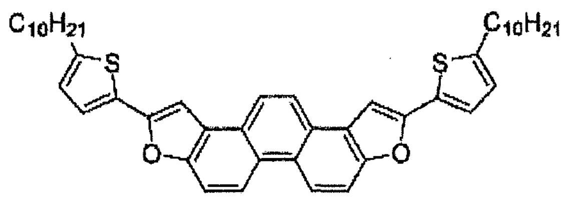

Compound 111

The compound represented by the formula (1) may have a repeating structure, or may have either low or high molecular weight. In the case where the compound represented by the formula (1) is a low molecular weight compound, it preferably has a molecular weight of 3,000 or less, more preferably 2,000 or less, further preferably 1,000 or less, and particularly preferably 850 or less. The molecular weight that is the upper limit or less is preferred since the compound has increased solubility in a solvent.

The molecular weight of the compound is preferably 400 or more, more preferably 450 or more, and further preferably 500 or more, from the standpoint of the stability of the film quality of the thin film.

In the case where the compound represented by the formula (1) is a high molecular weight compound having a repeating structure, a weight-average molecular weight is preferably 30,000 or more, more preferably 50,000 or more, and further preferably 100,000 or more. In the case where the compound represented by the formula (1) is a high molecular weight compound having a repeating structure, the weight-average molecular weight is preferably set to be equal to or more than the lower limit stated above, thereby enhancing the intermolecular interaction and achieving a high mobility.

Examples of the high molecular weight compound having a repeated structure include a $\pi$-conjugated polymer in which the compound represented by the formula (1) represents at least one of an arylene group and a heteroarylene group (thiophen or bithiophen) to express a repeating structure, and a pendant type polymer in which the compound

represented by the formula (1) is bonded to a polymer main chain via a side chain. The polymer main chain is preferably polyacrylate, polyvinyl or polysiloxane, and the side chain is preferably an alkylene group or a polyethylene oxide group.

The compound represented by the formula (1) may be synthesized with reference to the methods described in JP-A-2012-513459; WO 2011/46687; Journal of Chemical Research, Miniprint, 3, 601-635 (1991); Bull. Chem. Soc. Japan, 64, 3682-3686 (1991); and Tetrahedron Letters, 45, 2801-2803 (2004).

In the reaction of forming the compound of the invention, any reaction condition may be used. The reaction solvent used may be any solvent. An acid or a base is preferably used for promoting the ring-forming reaction, and particularly a base is preferably used. The optimum reaction condition may vary depending on the structure of the target phenanthrene derivative, and may be determined with reference to the specific reaction shown in the aforementioned literatures.

The synthesis intermediates having the various substituents may be synthesized by combining known reactions. The substituents may be introduced in any stage of the intermediates. The intermediates after synthesis is preferably purified by column chromatography, recrystallization or the like, and then purified by sublimation. The sublimation purification not only isolates organic impurities, but also effectively removes an inorganic salt, a residual solvent and the like.

<Structure of Organic Thin Film Transistor>

The organic thin film transistor of the invention has a semiconductor active layer containing the compound represented by the formula (1).

The organic thin film transistor of the invention may further contain other layers in addition to the semiconductor active layer.

The organic thin film transistor of the invention is preferably used as an organic field effect transistor (FET), and is more preferably used as an insulated gate FET, in which the gate and the channel are insulated from each other.

Preferred embodiments of the organic thin film transistor of the invention will be described below with reference to the drawings, but the invention is not limited to the embodiments.

(Laminated Structure)

The laminated structure of the organic field effect transistor is not particularly limited, and various known structures may be used.

One example of the structure of the organic thin film transistor of the invention is a bottom-gate top-contact structure having a substrate as the lowermost layer having disposed thereon an electrode, an insulating layer, a semiconductor active layer (organic semiconductor layer), and two electrodes, in this order. In this structure, the electrode on the upper surface of the substrate as the lowermost layer is provided on a part of the substrate, and the insulating layer is disposed to be in contact with the substrate in the portion other than the electrode. The two electrodes disposed on the upper surface of the semiconductor active layer are disposed to be separated from each other.

A structure of a bottom-gate top-contact device is shown in Fig. 1. Fig. 1 is a schematic illustration showing a cross sectional structure of one example of the organic thin film transistor of the invention. The organic thin film transistor shown in Fig. 1 has a substrate 11 disposed as the lowermost layer, an electrode 12 disposed on a part of the upper surface of the substrate 11, and an insulating layer 13 disposed to cover the electrode 12 and to be in contact with the substrate 11 in the portion other than the electrode 12. A semiconductor active layer 14 is provided on the upper surface of the insulating layer 13, and two electrodes 15a and 15b, which are separated from each other, are disposed on parts of the semiconductor active layer 14.

In the organic thin film transistor shown in Fig. 1, the electrode 12 is a gate, and the electrodes 15a and 15b each are a drain or a source. The organic thin film transistor shown in Fig. 1 is an insulated gate FET, in which the channel, which is an electric current path between the drain and the source, and the gate are insulated from each other.

Another example of the structure of the organic thin film transistor of the invention is a bottom-gate bottom-contact device.

A structure of a bottom-gate bottom-contact device is shown in Fig. 2. Fig. 2 is a schematic illustration showing a cross sectional structure of an organic thin film transistor that is produced as a substrate for measuring FET characteristics in the example of the invention. The organic thin film transistor shown in Fig. 2 has a substrate 31 disposed as the lowermost layer, an electrode 32 disposed on a part of the upper surface of the substrate 31, and an insulating layer 33 disposed to cover the electrode 32 and to be in contact with the substrate 31 in the portion other than the electrode 32. A semiconductor active layer 35 is provided on the upper surface of the insulating layer 33, and electrodes 34a and 34b are disposed under the semiconductor active layer 35.

In the organic thin film transistor shown in Fig. 2, the electrode 32 is a gate, and the electrodes 34a and 34b each are a drain or a source. The organic thin film transistor shown in Fig. 2 is an insulated gate FET, in which the channel, which is an electric current path between the drain and the source, and the gate are insulated from each other.

Other preferred examples of the structure of the organic thin film transistor of the invention include a top-gate top-contact device and a top-gate bottom-contact device, in which an insulator and a gate electrode are disposed above a semiconductor active layer.

(Thickness)

The organic thin film transistor of the invention preferably has a total thickness of the transistor, for example, of from 0.1 to 0.5 μm, in the case where a thinner transistor is demanded.

(Sealing)

For shielding the organic thin film transistor device from the air and water to enhance the storage stability of the organic thin film transistor device, the entire organic thin film transistor device may be sealed with a metallic sealing canister, an inorganic material, such as glass and silicon nitride, a polymer material, such as parylene, a low molecular weight material, and the like.

Preferred embodiments of the layers of the organic thin film transistor of the invention will be described below, but the invention is not limited to the embodiments.

<Substrate>

(Material)

The organic thin film transistor of the invention preferably contains a substrate.

The material for the substrate is not particularly limited, and known materials may be used. Examples of the material include a polyester film, such as polyethylene naphthoate (PEN) and polyethylene terephthalate (PET), a cycloolefin polymer film, a polycarbonate film, a triacetyl cellulose (TAC) film, a polyimide film, these polymer films having an ultrathin glass layer laminated thereon, ceramics, silicone, quartz, glass, and the like, and silicone is preferred.

<Electrode>

(Material)

The organic thin film transistor of the invention preferably contains an electrode.

Examples of the material for the electrode include known electroconductive materials, for example, a metal material, such as Cr, Al, Ta, Mo, Nb, Cu, Ag, Au, Pt, Pd, In, Ni and Nd, an alloy material of the metal materials, a carbon material, and an electroconductive polymer, which may be used without particular limitation.

(Thickness)

The thickness of the electrode is not particularly limited and is preferably from 10 to 50 nm.

The gate width (or the channel width) W and the gate length (or the channel length) L are not particularly limited, and the ratio W/L is preferably 10 or more, and more preferably 20 or more.

<Insulating Layer>

(Material)

The material for the insulating layer is not particularly limited as far as the necessary insulating effect is obtained, and examples thereof include silicon dioxide, silicon nitride, a fluorine polymer insulating material, such as PTFE and CYTOP, a polyester insulating material, a polycarbonate insulating material, an acrylic polymer insulating material, an epoxy resin insulating material, a polyimide insulating material, a polyvinylphenol resin insulating material, and a poly-p-xylylene resin insulating material.

The upper surface of the insulating layer may be surface-treated, and preferred examples thereof used include an insulating layer formed of silicon dioxide, the surface of which is surface-treated by coating hexamethyldisilazane (HMDS) or octadecyltrichlorosilane (OTS) thereon.

(Thickness)

The thickness of the insulating layer is not particularly limited, and in the case where a thin insulating layer is demanded, the thickness thereof is preferably from 10 to 400 nm, more preferably from 20 to 200 nm, and particularly preferably from 50 to 200 nm.

<Semiconductor Active Layer>

(Material)

The organic thin film transistor of the invention contains the compound represented by the formula (1), i.e., the compound of the invention, in the semiconductor active layer.
The semiconductor active layer may be a layer that is formed of the compound of the invention, or a layer containing a polymer binder described later in addition to the compound of the invention. The semiconductor active layer may contain a residual solvent used on forming the film.
The content of the polymer binder in the semiconductor active layer is not particularly limited, and the polymer binder is preferably used in a range of from 0 to 95% by mass, more preferably used in a range of from 10 to 90% by mass, further preferably used in a range of from 20 to 80% by mass, and particularly preferably used in a range of from 30 to 70% by mass.

(Thickness)

The thickness of the semiconductor active layer is not particularly limited, and in the case where a thin semiconductor active layer is demanded, the thickness thereof is preferably from 10 to 400 nm, more preferably from 10 to 200 nm, and particularly preferably from 10 to 100 nm.

[Organic Semiconductor Material for Non-light Emitting Organic Semiconductor Device]

The invention also relates to an organic semiconductor material for a non-light emitting organic semiconductor device containing the compound represented by the formula (1), i.e., the compound of the invention.

(Non-light Emitting Organic Semiconductor Device)

The non-light emitting organic semiconductor device referred herein means a device that is not intended to emit light. The non-light emitting organic semiconductor device is preferably a non-light emitting organic semiconductor device that uses an electronic element having a layer structure of thin films. The non-light emitting organic semiconductor device encompasses an organic thin film transistor, an organic photoelectric conversion device (such as a solid state imaging device for a photosensor, and a solar cell for energy conversion), a gas sensor, an organic rectifying device, an organic inverter, an information recording device, and the like. The organic photoelectric conversion device may be used for both a photosensor (i.e., a solid state imaging device) and energy conversion (i.e., a solar cell). Preferred examples of the device include an organic photoelectric conversion device and an organic thin film transistor, and more preferred examples thereof include an organic thin film transistor. Accordingly, the organic semiconductor device for a non-light emitting organic semiconductor device of the invention is preferably a material for an organic thin film transistor as described above.

(Organic Semiconductor Material)

The organic semiconductor material referred herein means an organic material that shows characteristics of a semiconductor. The organic semiconductor material includes a p-type (hole transporting) organic semiconductor, which shows conductivity with holes as a carrier, and an n-type (electron transporting) organic semiconductor, which shows conductivity with electrons as a carrier, as similar to a semiconductor material formed of an inorganic material.
The compound of the invention may be used as any of a p-type organic semiconductor material and an n-type organic semiconductor material, and is preferably used as a p-type organic semiconductor material. The flowability of a carrier in an organic semiconductor is shown by a carrier mobility $\mu$. The carrier mobility $\mu$ is preferably as large as possible, and is preferably $1 \times 10^{-3}$ $cm^2/Vs$ or more, more preferably $5 \times 10^{-3}$ $cm^2/Vs$ or more, particularly preferably $1 \times 10^{-2}$ $cm^2/Vs$ or more, further particularly preferably $1 \times 10^{-1}$ $cm^2/Vs$ or more, and still further particularly preferably 1 $cm^2/Vs$ or more. The carrier mobility $\mu$ may be obtained from the characteristics of a field effect transistor (FET) device produced or by a time-of-flight (TOF) measurement method.

[Organic Semiconductor Thin Film for Non-light Emitting Organic Semiconductor Device]

(Material)

The invention also relates to an organic semiconductor thin film for a non-light emitting organic semiconductor device containing the compound represented by the formula (1), i.e., the compound of the invention.

The organic semiconductor thin film for a non-light emitting organic semiconductor device of the invention contains the compound represented by the formula (1), i.e., the compound of the invention, and an embodiment thereof that contains no polymer binder is also preferred.

The organic semiconductor thin film for a non-light emitting organic semiconductor device of the invention may contain the compound represented by the formula (1), i.e., the compound of the invention, and a polymer binder.

Examples of the polymer binder include an insulating polymer, such as polystyrene, polycarbonate, polyarylate, polyester, polyamide, polyimide, polyurethane, polysiloxane, polysulfone, polymethyl methacrylate, polymethyl acrylate, cellulose, polyethylene and polypropylene, copolymers thereof, a photoconductive polymer, such as polyvinylcarbazole and polysilane, and an electroconductive polymer and a semiconductor polymer, such as polythiophene, polypyrrole, polyaniline and poly-p-phenylenevinylene.

The polymer binder may be used solely or as a combination of plural kinds thereof.

The organic semiconductor material and the polymer binder may be uniformly mixed, or a part or the whole thereof may be phase-separated, and from the standpoint of the charge mobility, such a structure that the organic semiconductor and the binder are phase-separated in the thickness direction in the film is most preferred since the charge migration of the organic semiconductor may not be inhibited by the binder.

Taking the mechanical strength of the thin film into consideration, a polymer binder having a high glass transition temperature is preferred, and taking the charge mobility into consideration, a polymer binder having a structure that contains no polar group, a photoconductive polymer, and an electroconductive polymer are preferred.

The amount of the polymer binder used is not particularly limited, and the polymer binder may be preferably used in a range of from 0 to 95% by mass, more preferably used in a range of from 10 to 90% by mass, further preferably used in a range of from 20 to 80% by mass, and particularly preferably used in a range of from 30 to 70% by mass, in the organic semiconductor thin film for a non-light emitting organic semiconductor device of the invention.

In the invention, an organic thin film having good film quality may be obtained by using the compound having the aforementioned structure. Specifically, the compound of the invention has good crystallinity to enable formation of a film having a sufficient thickness, and thus the organic semiconductor thin film for a non-light emitting organic semiconductor device of the invention thus obtained may have good quality.

(Film Forming Method)

The compound of the invention may be formed as a film on a substrate by any method.

On forming the film, the substrate may be heated or cooled, and the film quality and the molecular packing in the film may be controlled by changing the temperature of the substrate. The temperature of the substrate is not particularly limited, and is preferably in a range of from 0 to 200°C, more preferably in a range of from 15 to 100°C, and particularly preferably in a range of from 20 to 95°C.

On forming a film of the compound of the invention on a substrate, the film may be formed by a vacuum process or a solution process, both of which are preferred.

Specific examples of the film formation by a vacuum process include a physical vapor phase growing method, such as a vacuum vapor deposition method, a sputtering method, an ion plating method and a molecular beam epitaxy (MBE) method, and a chemical vapor deposition (CVD) method, such as plasma polymerization, and a vacuum vapor deposition method is preferably used.

The film formation by a solution process means a method, in which an organic compound is dissolved in a solvent capable of dissolving the same, and a film is formed by using the resulting solution. Specific examples thereof used include ordinary methods, for example, a coating method, such as a casting method, a dip coating method, a die coater method, a roll coater method, a bar coater method and a spin coating method, a printing method, such as an ink-jet method, a screen printing method, a gravure printing method, a flexography printing method, an offset printing method and a microcontact printing method, and a Langmuir-Blodgett (LB) method, and a casting method, a spin coating method, an ink-jet method, a gravure printing method, a flexography printing method, an offset printing method and a microcontact printing method are particularly preferably used.

The organic semiconductor thin film for a non-light emitting organic semiconductor device of the invention is preferably produced by a solution coating method. In the case where the organic semiconductor thin film for a non-light emitting organic semiconductor device of the invention contains a polymer binder, the thin film is preferably formed such a method that the material for forming the layer and the polymer binder are dissolved or dispersed in a suitable solvent

to prepare a coating liquid, which is then coated by various coating methods to form the thin film.

The coating solution for a non-light emitting organic semiconductor device of the invention capable of being used for film formation by a solution process will be described below.

[Coating Solution for Non-light Emitting Organic Semiconductor Device]

The invention also relates to a coating solution for a non-light emitting organic semiconductor device containing the compound represented by the formula (1), i.e., the compound of the invention.

In the case where the film is formed on a substrate by a solution process, the material for forming the layer may be dissolved or dispersed in a suitable organic solvent (for example, a hydrocarbon solvent, such as hexane, octane, decane, toluene, xylene, mesitylene, ethylbenzene, decalin and 1-methylnaphthalene, a ketone solvent, such as acetone, methyl ethyl ketone, methyl isobutyl ketone and cyclohexanone, a halogenated hydrocarbon solvent, such as dichloromethane, chloroform, tetrachloromethane, dichloroethane, trichloroethane, tetrachloroethane, chlorobenzene, dichlorobenzene and chlorotoluene, an ester solvent, such as ethyl acetate, butyl acetate and amyl acetate, an alcohol solvent, such asmethanol, propanol, butanol, pentanol, hexanol, cyclohexanol, methyl cellosolve, ethyl cellosolve and ethylene glycol, an ether solvent, such as dibutyl ether, tetrahydrofuran, dioxane and anisole, an amide or imide solvent, such as N,N-dimethylformamide, N,N-dimethylacetamide, 1-methyl-2-pyrrolidone and 1-methyl-2-imidazolidinone, a sulfoxide solvent, such as dimethylsulfoxide, and a nitrile solvent, such as acetonitrile) and/or water to prepare a coating liquid, which may be then coated by various coating methods to form the thin film. The solvent may be used solely or as a combination of plural kinds thereof. Among these, a hydrocarbon solvent, a halogenated hydrocarbon solvent and an ether solvent are preferred, toluene, xylene, mesitylene, tetralin, dichlorobenzene and anisole are more preferred, and toluene, xylene, tetralin and anisole are particularly preferred. The concentration of the compound represented by the formula (1) in the coating liquid is preferably from 0.1 to 80% by mass, more preferably from 0.1 to 10% by mass, and particularly preferably from 0.5 to 10% by mass, by which a film having an arbitrary thickness may be formed.

For forming a film by a solution process, it is necessary to dissolve the materials in the aforementioned solvent, but it is insufficient that the materials are simply dissolved in the solvent. In general, a material to be formed into a film by a vacuum process may be dissolved in a solvent in a certain extent. However, the solution process includes a step of evaporating the solvent to form a thin film, after coating the materials dissolved in a solvent, and most of materials that are not suitable for forming a film by a solution process have high crystallinity, and thus may be disadvantageously crystallized (agglomerated) in the step to fail to provide a favorable thin film. The compound represented by the formula (1) is advantageous also in such a point that the compound may not cause the disadvantageous crystallization (agglomeration).

As the coating solution for a non-light emitting organic semiconductor device of the invention, such an embodiment is also preferred that contains the compound represented by the formula (1), i.e., the compound of the invention, and contains no polymer binder.

The coating solution for a non-light emitting organic semiconductor device of the invention may contain the compound represented by the formula (1), i.e., the compound of the invention, and a polymer binder. In this case, the thin film may be formed in such a manner that the material for forming the layer and the polymer binder are dissolved or dispersed in the suitable solvent described above to prepare a coating liquid, which is then coated by various coating method to form the thin film. The polymer binder may be selected from those described above.

Example

The features of the invention will be described more specifically with reference to examples and comparative examples below. The materials, the amounts used, the ratios, the contents of processes, the procedures of processes, and the like shown in the examples may be appropriately changed unless they deviate the substance of the invention. Accordingly, the scope of the invention is not construed as being limited to the following examples.

[Example 1]

<Synthesis Example 1> Synthesis of Compound 1

The Compound 1 as the compound represented by the formula (1) was synthesized by the specific synthesis procedures shown by the following scheme.

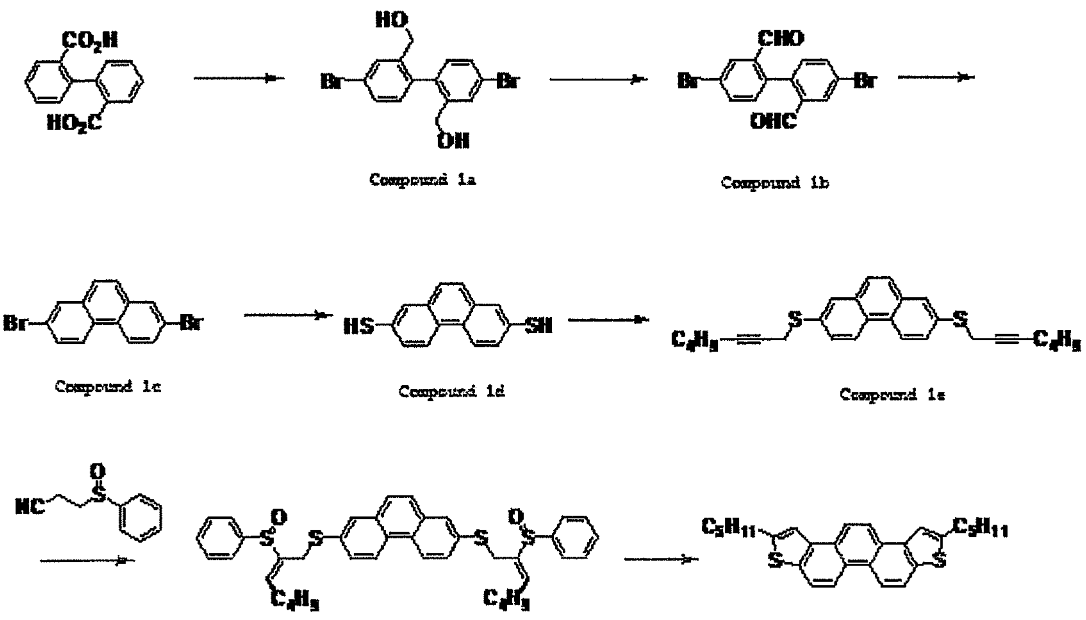

Compound 1a Compound 1b

Compound 1c Compound 1d Compound 1e

Compound 1f Compound 1

(Synthesis of Compounds 1a to 1d)

The Compounds 1a to 1d were synthesized according to a method described in Journal of Chemical Research, Miniprint, 1991, #3, pp.601-636.

(Synthesis of Compound 1e)

NMP solution (300 ml) of the Compound 1d (15 g), 1-bromohept-2-yne (23.7 g) and potassium carbonate (34.2 g) were stirred for at 100°C 6 hours. The reaction liquid was poured into a mixture of 1N hydrochloric acid aqueous solution and ethyl acetate. The organic layer was washed with a sodium chloride aqueous solution, dried over magnesium sulfate, and then concentrated under reduced pressure. The residue after the concentration was purified by silica gel column chromatography to provide a Compound 1e (20.1 g).

(Synthesis of Compound 1f)

The Compound 1e (20 g) and 1-cyano-2-phenysulfinylethane (2.4) were stirred in a nitrogen atmosphere at 110°C for 6 hours. The reaction mixture was purified by silica gel column chromatography to provide a Compound If (3.6 g).

(Synthesis of Compound 1)

A dry mesitylene solution (35 ml) of the Compound If (3.5 g) was stirred at 180°C for 2 days. The reaction liquid was concentrated under reduced pressure. The residue after the concentration was purified by silica gel column chromatography to provide a Compound 1 (0.57 g).

The compound was identified by elemental analysis, NMR and mass spectrum.

The other compounds represented by the formula (1) were synthesized in the similar manner as for the Compound 1.

Comparative compounds 1 to 5 used in a semiconductor active layer (organic semiconductor layer) of comparative devices were synthesized according to the methods described in the literatures. The structures of the comparative compounds 1 to 5 are shown below.

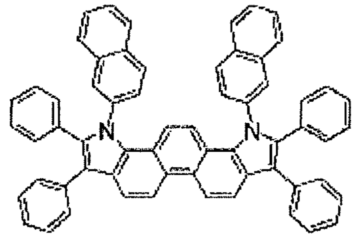

Comparative Compound 1 described in JP-A-2011-46687

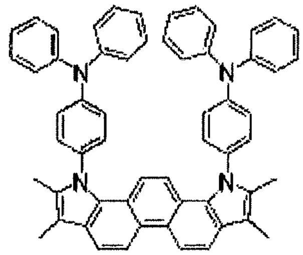

Comparative Compound 2 described in JP-A-2011-46687

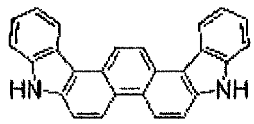

Comparative Compound 3 described in JP-T-2012-513459

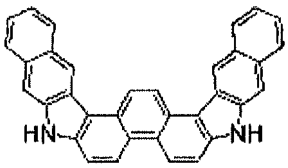

Comparative Compound 4 described in JP-T-2012-513459

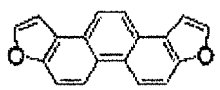

Comparative Compound 5

<Production and Evaluation of Devices>

All the materials used for producing devices were purified by sublimation, and were confirmed to have a purity (absorption intensity area ratio at 254 nm) of 99.5% or more by high-performance liquid chromatography (TSKgel ODS-100Z, available from Tosoh Corporation).

[Example 2]

<Formation of Semiconductor Active Layer (Organic Semiconductor Layer) only with Compound>

The compound of the invention or the comparative compound (1 mg each) and toluene (1 mL) were mixed and heated to 100°C to prepare a coating solution for a non-light emitting organic semiconductor device. The coating solution was cast on a substrate for measuring FET characteristics heated to 90°C under nitrogen atmosphere to form an organic

semiconductor thin film for a non-light emitting organic semiconductor device, thereby providing an organic thin film transistor device of Example 2 for measuring FET characteristics. The substrate for measuring FET characteristics used was a silicon substrate having a bottom-gate bottom-contact structure having chromium/gold electrodes (gate width W = 100 mm, gate length L = 100 μm) disposed in an interdigitated form as source and drain electrodes, and $SiO_2$ (thickness: 200 nm) as an insulating film (the schematic structural illustration shown in Fig. 2).

The FET characteristics of the organic thin film transistor device of Example 2 were evaluated in terms of the carrier mobility and the change in the threshold voltage after repeated driving by using a semiconductor parameter analyzer (4156C, produced by Agilent Technologies, Inc.) having a semi-automatic prober (AX-2000, produced by Vector Semiconductor Co., Ltd.) connected thereto under a normal pressure nitrogen atmosphere.

The results obtained are shown in Table below.

(a) Carrier Mobility

While applying a voltage of -80 V between the source electrode and the drain electrode of the organic thin film transistor device (FET device), the gate voltage was changed within a range of from 20 to -100 V, and the carrier mobility μ was calculated by the following expression showing the drain current $I_d$.

$$I_d = (W / 2L)\ \mu\ C_i\ (V_g - V_{th})^2$$

wherein L represents the gate length, W represents the gate width, $C_i$ represents the capacity of the insulating layer per unit area, $V_g$ represents the gate voltage, and $V_{th}$ represents the threshold voltage. A device that exhibited a carrier mobility of less than $1 \times 10^{-5}$ $cm^2/Vs$ was not subjected to the subsequent evaluation of (b) the change in the threshold voltage after repeated driving due to the too low property thereof.

(b) Change in Threshold Voltage after Repeated Driving

While applying a voltage of -80 V between the source electrode and the drain electrode of the organic thin film transistor device (FET device), the gate voltage was changed 100 times within a range of from 20 to -100 V, and the same measurement as in the measurement (a) above to evaluate the difference ($|V_1 - V_0|$) between the threshold voltage $V_0$ before repeated driving and the threshold voltage $V_1$ after repeated driving according to the following three grades. A smaller value thereof shows higher repeated driving stability of the device and thus is preferred.

A: $|V_1 - V_0| \leq 5$ V
B: 5 V < $|V_1 - V_0| \leq 10$ V
C: $|V_1 - V_0| > 10$ V

[Table 1]

| Device No. | Organic Semiconductor Material | Carrier Mobility ($cm^2/Vs$) | Change in Threshold Voltage after Repeated Driving | Note |
|---|---|---|---|---|
| Device 1 | Compound 81 | $4 \times 10^{-1}$ | A | invention |
| Device 2 | Compound 1 | $3 \times 10^{-1}$ | A | invention |
| Device 3 | Compound 5 | $3 \times 10^{-1}$ | A | invention |
| Device 4 | Compound 2 | $2 \times 10^{-1}$ | A | invention |
| Device 5 | Compound 7 | $5 \times 10^{-2}$ | A | invention |
| Device 6 | Compound 12 | $6 \times 10^{-2}$ | A | invention |
| Device 7 | Compound 13 | $1 \times 10^{-1}$ | B | invention |
| Device 8 | Compound 14 | $9 \times 10^{-2}$ | B | invention |
| Device 9 | Compound 17 | $2 \times 10^{-1}$ | A | invention |
| Device 10 | Compound 18 | $1 \times 10^{-1}$ | A | invention |

(continued)

| Device No. | Organic Semiconductor Material | Carrier Mobility ($cm^2/Vs$) | Change in Threshold Voltage after Repeated Driving | Note |
|---|---|---|---|---|
| Device 11 | Compound 31 | $7 \times 10^{-2}$ | A | invention |
| Device 12 | Compound 32 | $4 \times 10^{-2}$ | A | invention |
| Device 13 | Compound 49 | $6 \times 10^{-2}$ | A | invention |
| Device 14 | Compound 87 | $2 \times 10^{-1}$ | A | invention |
| Device 15 | Compound 88 | $2 \times 10^{-1}$ | A | invention |
| Device 16 | Compound 92 | $1 \times 10^{-1}$ | A | invention |
| Device 17 | Compound 85 | $1 \times 10^{-1}$ | A | invention |
| Device 18 | Compound 53 | $1 \times 10^{-1}$ | A | invention |
| Device 19 | Compound 54 | $9 \times 10^{-2}$ | A | invention |
| Device 20 | Compound 55 | $8 \times 10^{-2}$ | A | invention |
| Device 21 | Compound 93 | $5 \times 10^{-2}$ | A | invention |
| Device 22 | Compound 94 | $6 \times 10^{-2}$ | A | invention |
| Device 23 | Compound 95 | $4 \times 10^{-2}$ | A | invention |
| Device 24 | Compound 61 | $3 \times 10^{-2}$ | A | invention |
| Device 25 | Compound 62 | $1 \times 10^{-2}$ | A | invention |
| Device 26 | Compound 100 | $2 \times 10^{-1}$ | A | invention |
| Device 27 | Compound 72 | $1 \times 10^{-1}$ | A | invention |
| Device 28 | Compound 77 | $6 \times 10^{-3}$ | A | invention |
| Device 29 | Compound 78 | $4 \times 10^{-3}$ | A | invention |
| Comparative Device 1 | Comparative Compound 1 | $5 \times 10^{-5}$ | A | invention |
| Comparative Device 2 | Comparative Compound 2 | $< 1 \times 10^{-5}$ | - | comparison |
| Comparative Device 3 | Comparative Compound 3 | $< 1 \times 10^{-5}$ | - | comparison |
| Comparative Device 4 | Comparative Compound 4 | $8 \times 10^{-5}$ | C | comparison |
| Comparative Device 5 | Comparative Compound 5 | $2 \times 10^{-4}$ | C | comparison |

It was understood from Table 1 that the compounds of the invention had a good solubility in an organic solvent, and the organic thin film transistor devices using the compounds of the invention had a high carrier mobility. Accordingly, it was understood that the compound of the invention was favorably used as an organic semiconductor material for a non-light emitting organic semiconductor device.

On the other hand, the organic thin film transistor devices using the Comparative Compounds 1 to 5 had a low carrier mobility.

Moreover, it was understood that the organic thin film transistor devices using the compounds of the invention had a small change in the threshold voltage after repeated driving while the organic thin film transistor devices using the Comparative Compounds 4 and 5 had a large change in the threshold voltage after repeated driving.

[Example 3]

<Formation of Semiconductor Active Layer (Organic Semiconductor Layer) with both Compound and Binder>

Organic thin film transistor devices for measuring FET characteristics were produced in the same manner as in Example 2 except for using a coating solution prepared in such a manner that the compound of the invention or the comparative compound (1 mg each), 1 mg of PαMS (poly (α-methylstyrene), Mw: 300,000, produced by Sigma-Aldrich, Inc.) and toluene (1 mL) were mixed and heated to 100°C, and then evaluated in the same manner as in Example 2.
The results obtained are shown in Table below.

[Table 2]

| Device No. | Organic Semiconductor Material | Carrier Mobility ($cm^2/Vs$) | Change in Threshold Voltage after Repeated Driving | Note |
|---|---|---|---|---|
| Device 30 | Compound 1 | $8 \times 10^{-2}$ | A | invention |
| Device 31 | Compound 2 | $5 \times 10^{-2}$ | A | invention |
| Device 32 | Compound 13 | $9 \times 10^{-2}$ | A | invention |
| Device 33 | Compound 14 | $4 \times 10^{-2}$ | A | invention |
| Device 34 | Compound 31 | $8 \times 10^{-2}$ | A | invention |
| Device 35 | Compound 53 | $1 \times 10^{-1}$ | A | invention |
| Device 36 | Compound 72 | $7 \times 10^{-2}$ | A | invention |
| Comparative Device 6 | Comparative Compound 1 | $1 \times 10^{-5}$ | A | comparison |
| Comparative Device 7 | Comparative Compound 2 | $< 1 \times 10^{-5}$ | - | comparison |
| Comparative Device 8 | Comparative Compound 3 | $4 \times 10^{-5}$ | A | comparison |
| Comparative Device 9 | Comparative Compound 4 | $1 \times 10^{-4}$ | A | comparison |
| Comparative Device 10 | Comparative Compound 5 | $5 \times 10^{-4}$ | A | comparison |

It was understood from Table 2 that the compounds of the invention had a good solubility in an organic solvent, and the organic thin film transistor devices having a semiconductor active layer formed by using the compounds of the invention along with the binder had a high carrier mobility. Accordingly, it was understood that the compound of the invention was favorably used as an organic semiconductor material for a non-light emitting organic semiconductor device.
On the other hand, the organic thin film transistor devices having a semiconductor active layer formed by using the Comparative Compounds 1 to 5 along with the binder had a low carrier mobility.
Moreover, it was understood that the organic thin film transistor devices using the compounds of the invention had a small change in the threshold voltage after repeated driving.
It was understood from the observation with an optical microscope of the organic thin film transistor devices obtained in Example 3 that the thin films using PαMS as a binder all had considerably high smoothness and uniformity of the film.
It was understood from these results that the comparative devices having a semiconductor active layer formed with the composite system of the binder and the comparative compound had a considerably low carrier mobility, whereas the organic thin film transistor devices of the invention having a semiconductor active layer formed with both the compound of the invention and the binder had a good carrier mobility, a small change in the threshold voltage after repeated driving, and considerably high smoothness and uniformity of the film.

[Example 4]

<Formation of Semiconductor Active Layer (Organic Semiconductor Layer)>

A silicon wafer having a gate insulating film of $SiO_2$ (thickness: 370 nm) was subjected to a surface treatment with octyltrichlorosilane.

The compound of the invention or the comparative compound (1 mg each) and toluene (1 mL) were mixed and heated to 100°C to prepare a coating solution for a non-light emitting organic semiconductor device. The coating solution was cast on the octyltrichlorosilane-treated silicon wafer heated to 90°C under nitrogen atmosphere to form an organic semiconductor thin film for a non-light emitting organic semiconductor device.

On the surface of the thin film thus formed, gold was vapor-deposited through a mask to form source and drain electrodes, thereby providing an organic thin film transistor device having a bottom-gate top-contact structure having a gate width W of 5 mm and a gate length L of 80 μm (the schematic structural illustration shown in Fig. 1).

The FET characteristics of the organic thin film transistor device of Example 4 were evaluated in terms of the carrier mobility and the change in the threshold voltage after repeated driving by using a semiconductor parameter analyzer (4156C, produced by Agilent Technologies, Inc.) having a semi-automatic prober (AX-2000, produced by Vector Semiconductor Co., Ltd.) connected thereto under a normal pressure nitrogen atmosphere.

The results obtained are shown in Table 3 below.

[Table 3]

| Device No. | Organic Semiconductor Material | Carrier Mobility ($cm^2/Vs$) | Change in Threshold Voltage after Repeated Driving | Note |
|---|---|---|---|---|
| Device 37 | Compound 1 | $5 \times 10^{-1}$ | A | invention |
| Device 38 | Compound 3 | $2 \times 10^{-1}$ | A | invention |
| Device 39 | Compound 27 | $7 \times 10^{-2}$ | A | invention |
| Device 40 | Compound 31 | $1 \times 10^{-1}$ | A | invention |
| Device 41 | Compound 32 | $9 \times 10^{-2}$ | A | invention |
| Device 42 | Compound 53 | $4 \times 10^{-1}$ | A | invention |
| Device 43 | Compound 71 | $1 \times 10^{-1}$ | A | invention |
| Comparative Device 11 | Comparative Compound 1 | $3 \times 10^{-5}$ | A | comparison |
| Comparative Device 12 | Comparative Compound 2 | $< 1 \times 10^{-5}$ | - | comparison |
| Comparative Device 13 | Comparative Compound 3 | $4 \times 10^{-4}$ | C | comparison |
| Comparative Device 14 | Comparative Compound 4 | $6 \times 10^{-4}$ | C | comparison |
| Comparative Device 15 | Comparative Compound 5 | $1 \times 10^{-3}$ | C | comparison |

It was understood from Table 3 that the compounds of the invention had a good solubility in an organic solvent, and the organic thin film transistor devices using the compounds of the invention had a high carrier mobility. Accordingly, it was understood that the compound of the invention was favorably used as an organic semiconductor material for a non-light emitting organic semiconductor device.

On the other hand, the organic thin film transistor devices using the Comparative Compounds 1 to 5 had a low carrier mobility.

Moreover, it was understood that the organic thin film transistor devices using the compounds of the invention had a small change in the threshold voltage after repeated driving while the organic thin film transistor devices using the Comparative Compounds 3 to 5 had a large change in the threshold voltage after repeated driving.

Reference Signs List

11 substrate
12 electrode
13 insulating layer
14 semiconductor active layer (organic material layer or organic semiconductor layer)
15a, 15b electrode
31 substrate
32 electrode
33 insulating layer
34a, 34b electrode
35 semiconductor active layer (organic material layer or organic semiconductor layer)

## Claims

1. A compound represented by the following formula (1) :

Formula (1)

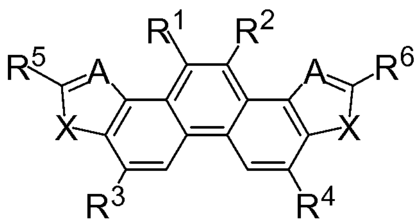

wherein X represents an S atom, an O atom or $NR^7$; A represents $CR^8$ or a nitrogen atom; **characterized in that**: $R^1$ to $R^8$ each independently represent a hydrogen atom or a substituent independently selected from a halogen atom, an alkyl group, an alkenyl group, an alkynyl group, an aryl group, a heterocyclic group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, an alkoxy- or aryloxycarbonylamino group, an alkyl- or arylsulfonylamino group, a mercapto group, an alkyl- or arylthio group, a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group and a hydrazino group, provided that at least one of $R^1$ to $R^8$ represents a substituent.

2. The compound according to Claim 1, wherein the compound represented by the formula (1) is a compound represented by the following formula (2-1), (2-2) or (2-3):

Formula (2-1)

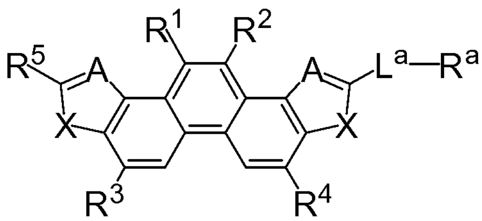

wherein in the formula (2-1), X represents an S atom, an O atom or $NR^7$; A each independently represents $CR^8$ or a nitrogen atom; $R^1$ to $R^5$, $R^7$ and $R^8$ each independently represent a hydrogen atom or a substituent independently

selected from a halogen atom, an alkyl group, an alkenyl group, an alkynyl group, an aryl group, a heterocyclic group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, an alkoxy- or aryloxycarbonylamino group, an alkyl- or arylsulfonylamino group, a mercapto group, an alkyl- or arylthio group, a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group and a hydrazino group, provided that $R^5$ is not represented by $-L^a-R^a$; $L^a$ each independently represents a divalent linking group represented by any one of the following formulae (L-1) to (L-12), or a divalent linking group consisting of 2 or more divalent linking groups each represented by any one of the following formulae (L-1) to (L-12) in which the 2 or more divalent linking groups are bonded sequentially; and $R^a$ each independently represents a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^a$ represents a substituted or unsubstituted trialkylsilyl group only when $L^a$ bonded to $R^a$ is a divalent linking group represented by the following formula (L-3), and $R^a$ represents a hydrogen atom only when $L^a$ bonded to $R^a$ is a divalent linking group represented by any one of the following formulae (L-1) to (L-3) and (L-10) to (L-12);

Formula (2-2)

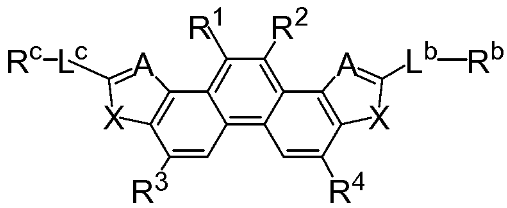

wherein in the formula (2-2), X represents an S atom, an O atom or $NR^7$; A represents $CR^8$ or a nitrogen atom; $R^1$ to $R^4$, $R^7$ and $R^8$ each independently represent a hydrogen atom or a substituent independently selected from a halogen atom, an alkyl group, an alkenyl group, an alkynyl group, an aryl group, a heterocyclic group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, an alkoxy- or aryloxycarbonylamino group, an alkyl- or arylsulfonylamino group, a mercapto group, an alkyl- or arylthio group, a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group and a hydrazino group; $L^b$ and $L^c$ each independently represent a divalent linking group represented by any one of the following formulae (L-1) to (L-12), or a divalent linking group consisting of 2 or more divalent linking groups each represented by any one of the following formulae (L-1) to (L-12) in which the 2 or more divalent linking groups are bonded sequentially; and $R^b$ and $R^c$ each independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^b$ and $R^c$ represent a substituted or unsubstituted trialkylsilyl group only when $L^b$ or $L^c$ bonded to $R^b$ or $R^c$ is a divalent linking group represented by the following formula (L-3), and $R^b$ and $R^c$ represent a hydrogen atom only when $L^b$ or $L^c$ bonded to $R^b$ or $R^c$ is a divalent linking group represented by any one of the following formulae (L-1) to (L-3) and (L-10) to (L-12);

Formula (2-3)

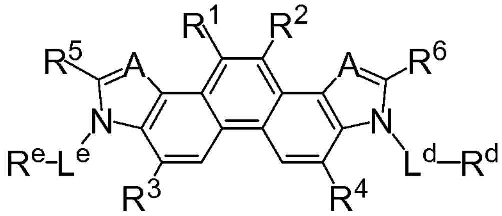

wherein in the formula (2-3), A represents $CR^8$ or a nitrogen atom; $R^1$ to $R^6$, and $R^8$ each independently represent a hydrogen atom or a substituent independently selected from a halogen atom, an alkyl group, an alkenyl group, an alkynyl group, an aryl group, a heterocyclic group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, an alkoxy- or aryloxycarbonylamino group, an alkyl- or arylsulfonylamino group, a mercapto group, an alkyl- or arylthio group, a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group and a hydrazino group; $L^d$ and $L^e$ each independently represent a divalent linking group represented by any one of the following formulae (L-1) to (L-3), (L-6), (L-7), and (L-9) to (L-12), or a divalent linking group consisting of 2 or more divalent linking groups each represented by any one of the following formulae (L-1) to (L-12) in which the 2 or more divalent linking groups are bonded sequentially; and $R^d$ and $R^e$ each independently represent a hydrogen atom, a substituted or unsubstituted alkyl group, an oligooxyethylene group having a repeating number of an oxyethylene unit of 2 or more, an oligosiloxane group having 2 or more silicon atoms, or a substituted or unsubstituted trialkylsilyl group, provided that $R^d$ and $R^e$ represent a substituted or unsubstituted trialkylsilyl group only when $L^d$ or $L^e$ bonded to $R^d$ or $R^e$ is a divalent linking group represented by the following formula (L-3), and $R^d$ and $R^e$ represent a hydrogen atom only when $L^d$ or $L^e$ bonded to $R^d$ or $R^e$ is a divalent linking group represented by any one of the following formulae (L-1) to (L-3) and (L-10) to (L-12); and

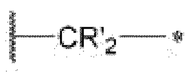 (L-1)

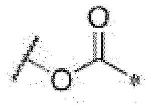 (L-8)

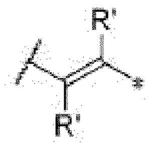 (L-2)

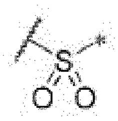 (L-9)

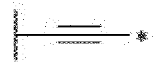 (L-3)

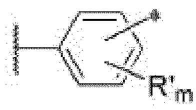 (L-10)

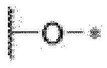 (L-4)

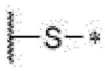 (L-5)

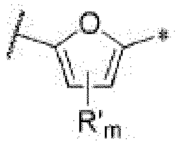 (L-11)

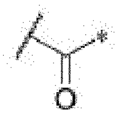 (L-6)

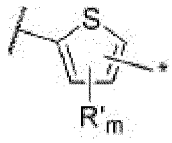 (L-12)

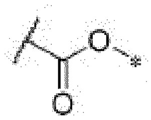 (L-7)

wherein in the formulae (L-1) to (L-12), the wavy line represents a position bonded to the phenanthrodifuran, phenanthrodithiphen or phenanthrodipyrrole skeleton, and * represents a position bonded to any one of $R^a$ to $R^e$; in the formula (L-10), m represents 4; in the formulae (L-11) and (L-12), m represents 2; in the formulae (L-1), (L-2), (L-10), (L-11) and (L-12), R' each independently represents a hydrogen atom or a substituent; and in the formulae (L-1) and (L-2), R' each independently may form a condensed ring with $R^a$ to $R^e$ each bonded to $L^a$ to $L^e$.

**3.** The compound according to Claim 1 or 2, wherein A in the formula (1), (2-1), (2-2) or (2-3) each independently represents $CR^8$ provided that $R^8$ represents a hydrogen atom or a substituent independently selected from a halogen atom, an alkyl group, an alkenyl group, an alkynyl group, an aryl group, a heterocyclic group, a cyano group, a hydroxyl group, a nitro group, an acyl group, an alkoxy group, an aryloxy group, a silyloxy group, a heterocyclic oxy

group, an acyloxy group, a carbamoyloxy group, an amino group, an acylamino group, an aminocarbonylamino group, an alkoxy- or aryloxycarbonylamino group, an alkyl- or arylsulfonylamino group, a mercapto group, an alkyl- or arylthio group, a heterocyclic thio group, a sulfamoyl group, a sulfo group, an alkyl- or arylsulfinyl group, an alkyl- or arylsulfonyl group, an alkyl- or aryloxycarbonyl group, a carbamoyl group, an aryl- or heterocyclic azo group, an imide group, a phosphino group, a phosphinyl group, a phosphinyloxy group, a phosphinylamino group, a phosphono group, a silyl group and a hydrazino group.

**4.** A compound according to any preceding claim, wherein A in the formula (1), (2-1), (2-2) or (2-3) is a group having a linking chain length of 0.37 nm (3.7 Å) or less.

**5.** The compound according to Claim 2, wherein all of $L^a$ to $L^e$ in the formula (2-1), (2-2) or (2-3) each represent a divalent linking group represented by any one of the formulae (L-1) to (L-5), (L-10), (L-11) and (L-12), or a divalent linking group consisting of 2 or more divalent linking groups each represented by any one of the formulae (L-1) to (L-5), (L-10), (L-11) and (L-12) in which the 2 or more divalent linking groups are bonded sequentially.

**6.** A compound according to Claim 2, wherein all of $L^a$ to $L^c$ in the formula (2-1) or (2-2) each represent a divalent linking group represented by any one of the formulae (L-1), (L-3), (L-10) and (L-12).

**7.** A compound according to Claim 2, wherein $L^d$ to $L^e$ in the formula (2-3) each independently represent a divalent linking group represented by any one of the formulae (L-10), (L-11) and (L-12).

**8.** The compound according to Claim 2, wherein all of $R^a$ to $R^e$ in the formula (2-1), (2-2) or (2-3) each represent a substituted or unsubstituted alkyl group.

**9.** The compound according to any preceding claim, wherein X represents an S atom or an O atom.

**10.** An organic semiconductor material for a non-light emitting organic semiconductor device, containing the compound as defined in any preceding Claim.

**11.** An organic thin film transistor, containing the compound as defined in any of Claims 1 to 9.

**12.** A coating solution for a non-light emitting organic semiconductor device containing the compound as defined in any of Claims 1 to 9.

**13.** A coating solution according to Claim 12, further containing a polymer binder.

**14.** An organic semiconductor thin film for a non-light emitting organic semiconductor device containing the compound as defined in any of Claims 1 to 9.

**15.** An organic semiconductor thin film according to Claim 14, further containing a polymer binder.

## Patentansprüche

**1.** Verbindung, dargestellt durch die folgende Formel (1):

Formel (1)

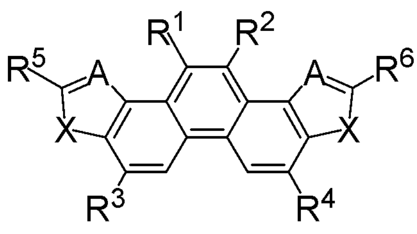

worin X ein S-Atom, ein O-Atom oder $NR^7$ darstellt; A $CR^8$ oder ein Stickstoffatom darstellt; **dadurch gekennzeich-**

**net, dass**:

$R^1$ bis $R^8$ jeweils unabhängig ein Wasserstoffatom oder einen Substituenten darstellen, der unabhängig ausgewählt ist aus einem Halogenatom, einer Alkylgruppe, einer Alkenylgruppe, einer Alkinylgruppe, einer Arylgruppe, einer heterocyclischen Gruppe, einer Cyanogruppe, einer Hydroxylgruppe, einer Nitrogruppe, einer Acylgruppe, einer Alkoxygruppe, einer Aryloxygruppe, einer Silyloxygruppe, einer heterocyclischen Oxygruppe, einer Acyloxygruppe, einer Carbamoyloxygruppe, einer Aminogruppe, einer Acylaminogruppe, einer Aminocarbonylaminogruppe, einer Alkoxy- oder Aryloxycarbonylaminogruppe, einer Alkyl- oder Arylsulfonylaminogruppe, einer Mercaptogruppe, einer Alkyl- oder Arylthiogruppe, einer heterocyclischen Thiogruppe, einer Sulfamoylgruppe, einer Sulfogruppe, einer Alkyl- oder Arylsulfinylgruppe, einer Alkyl- oder Arylsulfonylgruppe, einer Alkyl- oder Aryloxycarbonylgruppe, einer Carbamoylgruppe, einer Aryl- oder heterocyclischen Azogruppe, einer Imidgruppe, einer Phosphingruppe, einer Phosphinylgruppe, einer Phosphinyloxygruppe, einer Phosphinylaminogruppe, einer Phosphongruppe, einer Silylgruppe und einer Hydrazingruppe, mit der Maßgabe, dass zumindest eines von $R^1$ bis $R^8$ einen Substituent darstellt.

**2.** Verbindung gemäß Anspruch 1, worin die durch die Formel (1) dargestellte Verbindung eine durch die folgende Formel (2-1), (2-2) oder (2-3) dargestellte Verbindung ist:

Formel (2-1)

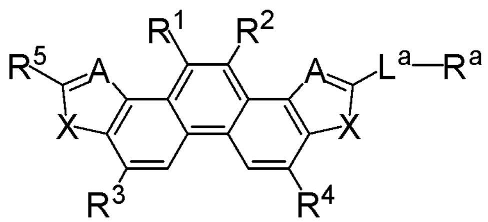

Worin in der Formel (2-1) X ein S-Atom, ein O-Atom oder $NR^7$ darstellt; A jeweils unabhängig $CR^8$ oder ein Stickstoffatom darstellt; $R^1$ bis $R^5$, $R^7$ und $R^8$ jeweils unabhängig ein Wasserstoffatom oder einen Substituenten darstellen, der unabhängig ausgewählt ist aus einem Halogenatom, einer Alkylgruppe, einer Alkenylgruppe, einer Alkinylgruppe, einer Arylgruppe, einer heterocyclischen Gruppe, einer Cyanogruppe, einer Hydroxylgruppe, einer Nitrogruppe, einer Acylgruppe, einer Alkoxygruppe, einer Aryloxygruppe, einer Silyloxygruppe, einer heterocyclischen Oxygruppe, einer Acyloxygruppe, einer Carbamoyloxygruppe, einer Aminogruppe, einer Acylaminogruppe, einer Aminocarbonylaminogruppe, einer Alkoxy- oder Aryloxycarbonylaminogruppe, einer Alkyl- oder Arylsulfonylaminogruppe, einer Mercaptogruppe, einer Alkyl- oder Arylthiogruppe, einer heterocyclischen Thiogruppe, einer Sulfamoylgruppe, einer Sulfogruppe, einer Alkyl- oder Arylsulfinylgruppe, einer Alkyl- oder Arylsulfonylgruppe, einer Alkyl- oder Aryloxycarbonylgruppe, einer Carbamoylgruppe, einer Aryl- oder heterocyclischen Azogruppe, einer Imidgruppe, einer Phosphingruppe, einer Phosphinylgruppe, einer Phosphinyloxygruppe, einer Phosphinylaminogruppe, einer Phosphongruppe, einer Silylgruppe und einer Hydrazingruppe, mit der Maßgabe, dass $R^5$ nicht durch $L^a$-$R^a$ dargestellt wird; $L^a$ jeweils unabhängig eine divalente Verknüpfungsgruppe, dargestellt durch irgendeine der folgenden Formeln (L-1) bis (L-12), oder eine divalente Verknüpfungsgruppe, die aus 2 oder mehr divalenten Verknüpfungsgruppen besteht, die jeweils durch irgendeine der folgenden Formeln (L-1) bis (L-12) dargestellt werden, darstellt, worin die 2 oder mehr divalenten Verknüpfungsgruppen sequentiell gebunden sind; und $R^a$ jeweils unabhängig ein Wasserstoffatom, eine substituierte oder unsubstituierte Alkylgruppe, eine Oligooxyethylengruppe mit einer Wiederholungszahl der Oxyethyleneinheit von 2 oder mehr, eine Oligosiloxangruppe mit 2 oder mehr Siliciumatomen oder eine substituierte oder unsubstituierte Trialkylsilylgruppe darstellt, mit der Maßgabe, dass $R^a$ nur eine substituierte oder unsubstituierte Trialkylsilylgruppe darstellt, wenn $L^a$, das an $R^a$ gebunden ist, eine divalente Verknüpfungsgruppe ist, die durch die folgende Formel (L-3) dargestellt ist, und $R^a$ nur ein Wasserstoffatom darstellt, wenn $L^a$, das an $R^a$ gebunden ist, eine divalente Verknüpfungsgruppe ist, die durch irgendeine der folgenden Formeln (L-1) bis (L-3) und (L-10) bis (L-12) dargestellt wird;

Formel (2-2)

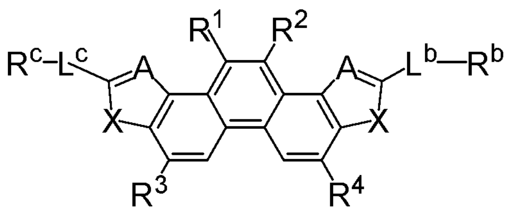

worin in der Formel (2-2) X ein S-Atom, ein O-Atom oder $NR^7$ darstellt; A $CR^8$ oder ein Stickstoffatom darstellt; $R^1$ bis $R^4$, $R^7$ und $R^8$ jeweils unabhängig ein Wasserstoffatom oder einen Substituenten darstellen, der unabhängig ausgewählt ist aus einem Halogenatom, einer Alkylgruppe, einer Alkenylgruppe, einer Alkinylgruppe, einer Arylgruppe, einer heterocyclischen Gruppe, einer Cyanogruppe, einer Hydroxylgruppe, einer Nitrogruppe, einer Acylgruppe, einer Alkoxygruppe, einer Aryloxygruppe, einer Silyloxygruppe, einer heterocyclischen Oxygruppe, einer Acyloxygruppe, einer Carbamoyloxygruppe, einer Aminogruppe, einer Acylaminogruppe, einer Aminocarbonylaminogruppe, einer Alkoxy- oder Aryloxycarbonylaminogruppe, einer Alkyl- oder Arylsulfonylaminogruppe, einer Mercaptogruppe, einer Alkyl- oder Arylthiogruppe, einer heterocyclischen Thiogruppe, einer Sulfamoylgruppe, einer Sulfogruppe, einer Alkyl- oder Arylsulfinylgruppe, einer Alkyl- oder Arylsulfonylgruppe, einer Alkyl- oder Aryloxycarbonylgruppe, einer Carbamoylgruppe, einer Aryl- oder heterocyclischen Azogruppe, einer Imidgruppe, einer Phosphingruppe, einer Phosphinylgruppe, einer Phosphinyloxygruppe, einer Phosphinylaminogruppe, einer Phosphongruppe, einer Silylgruppe und einer Hydrazingruppe, $L^b$ und $L^c$ jeweils unabhängig eine divalente Verknüpfungsgruppe, dargestellt durch irgendeine der folgenden Formeln (L-1) bis (L-12), oder eine divalente Verknüpfungsgruppe, die aus 2 oder mehr divalenten Verknüpfungsgruppen besteht, die jeweils durch irgendeine der folgenden Formeln (L-1) bis (L-12) dargestellt werden, darstellen, worin die 2 oder mehr divalenten Verknüpfungsgruppen sequentiell gebunden sind, darstellen; und $R^b$ und $R^c$ jeweils unabhängig ein Wasserstoffatom, eine substituierte oder unsubstituierte Alkylgruppe, eine Oligooxyethylengruppe mit einer Wiederholungszahl der Oxyethyleneinheit von 2 oder mehr, eine Oligosiloxangruppe mit 2 oder mehr Siliciumatomen oder eine substituierte oder unsubstituierte Trialkylsilylgruppe darstellen, mit der Maßgabe, dass $R^b$ und $R^c$ nur eine substituierte oder unsubstituierte Trialkylsilylgruppe darstellen, wenn $L^b$ oder $L^c$, gebunden an $R^b$ oder $R^c$, eine durch die folgende Formel (L-3) dargestellte divalente Verknüpfungsgruppe ist, und $R^b$ und $R^c$ nur ein Wasserstoffatom darstellen, wenn $L^b$ oder $L^c$, gebunden an $R^b$ oder $R^c$, eine divalente Verknüpfungsgruppe ist, die durch irgendeine der folgenden Formeln (L-1) bis (L-3) und (L-10) bis (L-12) dargestellt wird;

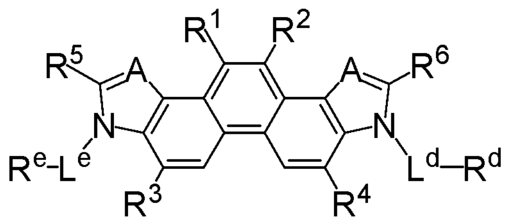

worin in der Formel (2-3) A $CR^8$ oder ein Stickstoffatom darstellt; $R^1$ bis $R^6$ und $R^8$ jeweils unabhängig ein Wasserstoffatom oder einen Substituenten darstellen, der unabhängig ausgewählt ist aus einem Halogenatom, einer Alkylgruppe, einer Alkenylgruppe, einer Alkinylgruppe, einer Arylgruppe, einer heterocyclischen Gruppe, einer Cyanogruppe, einer Hydroxylgruppe, einer Nitrogruppe, einer Acylgruppe, einer Alkoxygruppe, einer Aryloxygruppe, einer Silyloxygruppe, einer heterocyclischen Oxygruppe, einer Acyloxygruppe, einer Carbamoyloxygruppe, einer Aminogruppe, einer Acylaminogruppe, einer Aminocarbonylaminogruppe, einer Alkoxy- oder Aryloxycarbonylaminogruppe, einer Alkyl- oder Arylsulfonylaminogruppe, einer Mercaptogruppe, einer Alkyl- oder Arylthiogruppe, einer heterocyclischen Thiogruppe, einer Sulfamoylgruppe, einer Sulfogruppe, einer Alkyl- oder Arylsulfinylgruppe, einer Alkyl- oder Arylsulfonylgruppe, einer Alkyl- oder Aryloxycarbonylgruppe, einer Carbamoylgruppe, einer Aryl- oder heterocyclischen Azogruppe, einer Imidgruppe, einer Phosphingruppe, einer Phosphinylgruppe, einer Phosphinyloxygruppe, einer Phosphinylaminogruppe, einer Phosphongruppe, einer Silylgruppe und einer Hydrazingruppe, $L^d$ und $L^e$ jeweils unabhängig eine divalente Verknüpfungsgruppe, dargestellt durch irgendeine der folgenden Formeln (L-1) bis (L-3), (L-6), (L-7) und (L-9) bis (L-12), oder eine divalente Verknüpfungsgruppe, bestehend aus 2 oder mehr divalenten Verknüpfungsgruppen, die jeweils durch irgendeine der folgenden Formeln (L-1) bis (L-12) dargestellt werden, worin die 2 oder mehr divalenten Verknüpfungsgruppen sequentiell gebunden sind, darstellen; und

$R^d$ und $R^e$ jeweils unabhängig ein Wasserstoffatom, eine substituierte oder unsubstituierte Alkylgruppe, eine Oligooxyethylengruppe mit einer Wiederholungszahl der Oxyethyleneinheit von 2 oder mehr, eine Oligosiloxangruppe mit 2 oder mehr Siliciumatomen oder eine substituierte oder unsubstituierte Trialkylsilylgruppe darstellen, mit der Maßgabe, dass $R^d$ und $R^e$ nur eine substituierte oder unsubstituierte Trialkylsilylgruppe darstellen, wenn $L^d$ oder $L^e$, gebunden an $R^d$ oder $R^e$, eine durch die folgende Formel (L-3) dargestellte divalente Verknüpfungsgruppe ist, und $R^d$ und $R^e$ nur ein Wasserstoffatom darstellen, wenn $L^d$ oder $L^e$, gebunden an $R^d$ oder $R^e$, eine divalente Verknüpfungsgruppe ist, dargestellt durch irgendeine der folgenden Formeln (L-1) bis (L-3) und (L-10) bis (L-12); und

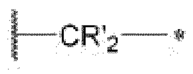 (L-1)

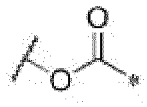 (L-8)

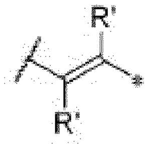 (L-2)

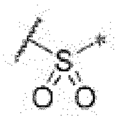 (L-9)

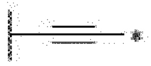 (L-3)

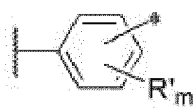 (L-10)

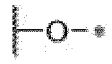 (L-4)

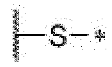 (L-5)

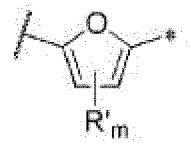 (L-11)

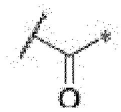 (L-6)

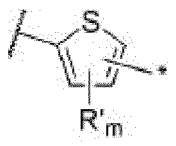 (L-12)

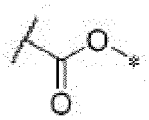 (L-7)

worin in den Formeln (L-1) bis (L-12) die gewellte Line eine Position darstellt, die an das Phenanthrodifuran-, Phenanthrodithiphen- oder Phenanthrodipyrrol-Gerüst gebunden ist, und * eine Position darstellt, die an irgendeines von $R^a$ bis $R^e$ gebunden ist; in der Formel (L-10), m 4 darstellt; in den Formeln (L-11) und (L-12) m 2 darstellt; in den Formeln (L-1), (L-2), (L-10), (L-11) und (L-12) R' jeweils unabhängig ein Wasserstoffatom oder einen Substituenten darstellt; und in den Formeln (L-1) und (L-2) R' jeweils unabhängig einen kondensierten Ring mit $R^a$ bis $R^e$ bilden kann, jeweils gebunden an $R^a$ bis $R^e$.

3. Verbindung gemäß Anspruch 1 oder 2, worin A in der Formel (1), (2-1), (2-2) oder (2-3) jeweils $CR^8$ darstellt, mit der Maßgabe, dass $R^8$ ein Wasserstoffatom oder einen Substituenten darstellt, der unabhängig ausgewählt ist aus einem Halogenatom, einer Alkylgruppe, einer Alkenylgruppe, einer Alkinylgruppe, einer Arylgruppe, einer heterocyclischen Gruppe, einer Cyanogruppe, einer Hydroxylgruppe, einer Nitrogruppe, einer Acylgruppe, einer Alkoxygruppe, einer Aryloxygruppe, einer Silyloxygruppe, einer heterocyclischen Oxygruppe, einer Acyloxygruppe, einer Carbamoyloxygruppe, einer Aminogruppe, einer Acylaminogruppe, einer Aminocarbonylaminogruppe, einer Alkoxy- oder Aryloxycarbonylaminogruppe, einer Alkyl- oder Arylsulfonylaminogruppe, einer Mercaptogruppe, einer Alkyl- oder Arylthiogruppe, einer heterocyclischen Thiogruppe, einer Sulfamoylgruppe, einer Sulfogruppe, einer Alkyl- oder Arylsulfinylgruppe, einer Alkyl- oder Arylsulfonylgruppe, einer Alkyl- oder Aryloxycarbonylgruppe, einer Carbamoylgruppe, einer Aryl- oder heterocyclischen Azogruppe, einer Imidgruppe, einer Phosphingruppe, einer Phosphinylgruppe, einer Phosphinyloxygruppe, einer Phosphinylaminogruppe, einer Phosphongruppe, einer Silylgruppe und einer Hydrazingruppe.

4. Verbindung gemäß irgendeinem vorstehenden Anspruch, worin A in den Formeln (1), (2-1), (2-2) oder (2-3) eine Gruppe ist die eine Verknüpfungs-Kettenlänge von 0,37 nm (3,7 Ä) oder kleiner aufweist.

5. Verbindung gemäß Anspruch 2, worin alle von $L^a$ bis $L^e$ in der Formel (2-1), (2-2) oder (2-3) jeweils eine divalente

Verknüpfungsgruppe, die durch irgendeine der Formeln (L-1) bis (L-5), (L-10), (L-11) und (L-12) dargestellt werden, oder eine divalente Verknüpfungsgruppe, die aus 2 oder mehr divalenten Verknüpfungsgruppen besteht, die jeweils durch irgendeine der Formeln (L-1) bis (L-5), (L-10), (L-11) und (L-12) dargestellt werden, worin die 2 oder mehr divalenten Verknüpfungsgruppen sequentiell gebunden sind, darstellen.

**6.** Verbindung gemäß Anspruch 2, worin alle von $L^a$ bis $L^c$ in der Formel (2-1) oder (2-2) jeweils eine divalente Verknüpfungsgruppe darstellen, die durch irgendeine der Formeln (L-1), (L-3), (L-10) und (L-12) dargestellt wird.

**7.** Verbindung gemäß Anspruch 2, worin $L^d$ und $L^e$ in der Formel (2-3) jeweils eine divalente Verknüpfungsgruppe darstellen, die durch irgendeine der Formeln (L-10), (L-11) und (L-12) dargestellt wird.

**8.** Verbindung gemäß Anspruch 2, worin alle von $R^a$ und $R^e$ in der Formel (2-1), (2-2) oder (2-3) jeweils eine substituierte oder unsubstituierte Alkylgruppe darstellen.

**9.** Verbindung gemäß irgendeinem vorstehenden Anspruch, worin X ein S-Atom oder ein O-Atom darstellt.

**10.** Organisches Halbleitermaterial für eine nicht-lichtemittierende organische Halbleitervorrichtung, enthaltend die Verbindung, wie sie in irgendeinem vorstehenden Anspruch definiert ist.

**11.** Organischer Dünnfilmtransistor, enthaltend die wie in irgendeinem der Ansprüche 1 bis 9 definierte Verbindung.

**12.** Beschichtungslösung für eine nicht-lichtemittierende organische Halbleitervorrichtung, enthaltend die wie in irgendeinem der Ansprüche 1 bis 9 definierte Verbindung.

**13.** Beschichtungslösung gemäß Anspruch 12, ferner enthaltend ein Polymer-Bindemittel.

**14.** Organischer Halbleiter-Dünnfilm für eine nicht-lichtemittierende organische Halbleitervorrichtung, enthaltend die wie in irgendeinem der Ansprüche 1 bis 9 definierte Verbindung.

**15.** Organischer Halbleiter-Dünnfilm gemäß Anspruch 14, ferner enthaltend ein Polymer-Bindemittel.

## Revendications

**1.** Composé représenté par la formule (1) suivante :

Formule (1)

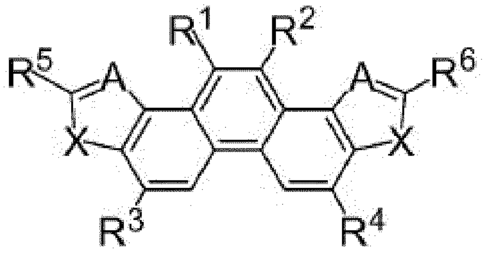

dans laquelle X représente un atome S, un atome O ou $NR^7$ ; A représente $CR^8$ ou un atome d'azote ; **caractérisé en ce que** :

$R^1$ à $R^8$ représentent chacun indépendamment
un atome d'hydrogène ou un substituant sélectionné indépendamment parmi un atome d'halogène, un groupe alkyle, un groupe alcényle, un groupe alcynyle, un groupe aryle, un groupe hétérocyclique, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un groupe carbamoyloxy, un groupe amino, un groupe acylamino, un groupe aminocarbonylamino, un groupe alcoxy- ou aryloxy-carbonylamino, un groupe alkyl- ou aryl-sulfonylamino, un groupe mercapto, un groupe alkyl- ou aryl-thio, un groupe thio hétérocyclique, un groupe sulfamoyle, un groupe sulfo, un groupe alkyl- ou aryl-sulfinyle, un groupe alkyl- ou aryl-sulfonyle, un groupe

alkyl- ou aryl-oxycarbonyle, un groupe carbamoyle, un groupe aryl- ou hétérocyclique azo, un groupe imide, un groupe phosphino, un groupe phosphinyle, un groupe phosphinyloxy, un groupe phosphinylamino, un groupe phosphono, un groupe silyle et un groupe hydrazino, à condition qu'au moins l'un parmi $R^1$ à $R^8$ représente un substituant.

2. Composé selon la revendication 1, dans lequel le composé représenté par la formule (1) est un composé représenté par la formule (2-1), (2-2) ou (2-3) suivante :

Formule (2-1)

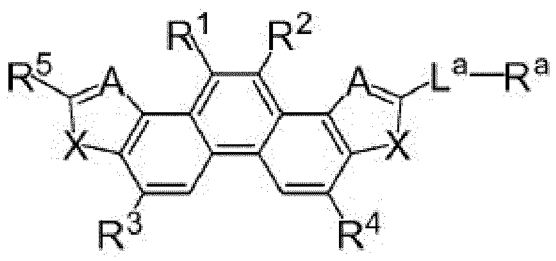

dans laquelle dans la formule (2-1), X représente un atome S, un atome O ou $NR^7$ ; les A représentent chacun indépendamment $CR^8$ ou un atome d'azote ; $R^1$ à $R^5$, $R^7$ et $R^8$ représentent chacun indépendamment un atome d'hydrogène ou un substituant sélectionné indépendamment parmi un atome d'halogène, un groupe alkyle, un groupe alcényle, un groupe alcynyle, un groupe aryle, un groupe hétérocyclique, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un groupe carbamoyloxy, un groupe amino, un groupe acylamino, un groupe aminocarbonylamino, un groupe alcoxy- ou aryloxy-carbonylamino, un groupe alkyl- ou aryl-sulfonylamino, un groupe mercapto, un groupe alkyl- ou aryl-thio, un groupe thio hétérocyclique, un groupe sulfamoyle, un groupe sulfo, un groupe alkyl- ou aryl-sulfinyle, un groupe alkyl- ou aryl-sulfonyle, un groupe alkyl- ou aryl-oxycarbonyle, un groupe carbamoyle, un groupe aryl- ou hétérocyclique azo, un groupe imide, un groupe phosphino, un groupe phosphinyle, un groupe phosphinyloxy, un groupe phosphinylamino, un groupe phosphono, un groupe silyle et un groupe hydrazino, à condition que $R^5$ ne soit pas représenté par $-L^a-R^a$ ; $L^a$ représente chacun indépendamment un groupe de liaison divalent représenté par l'une quelconque des formules (L-1) à (L-12) suivantes, ou un groupe de liaison divalent consistant en 2 ou plus de 2 groupes de liaison divalents représentés chacun par l'une quelconque des formules (L-1) à (L-12) suivantes dans lesquelles les 2 ou plus de 2 groupes de liaison divalents sont liés de manière séquentielle; et $R^a$ représente chacun indépendamment un atome d'hydrogène, un groupe alkyle substitué ou non substitué, un groupe oligooxyéthylène présentant un nombre répétitif d'un motif oxyéthylène égal à 2 ou plus, un groupe oligosiloxane présentant 2 atomes de silicium ou plus, ou un groupe trialkylsilyle substitué ou non substitué, à condition que $R^a$ représente un groupe trialkylsilyle substitué ou non substitué uniquement quand $L^a$ lié à $R^a$ est un groupe de liaison divalent représenté par la formule (L-3) suivante, et $R^a$ représente un atome d'hydrogène uniquement quand $L^a$ lié à $R^a$ est un groupe de liaison divalent représenté par l'une quelconque des formules (L-1) à (L-3) et (L-10) à (L-12) suivantes ;

Formule (2-2)

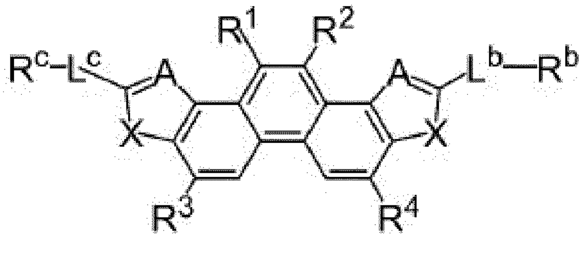

dans laquelle dans la formule (2-2), X représente un atome S, un atome O ou $NR^7$ ; A représente $CR^8$ ou un atome d'azote ; $R^1$ à $R^4$, $R^7$ et $R^8$ représentent chacun indépendamment un atome d'hydrogène ou un substituant sélectionné indépendamment parmi un atome d'halogène, un groupe alkyle, un groupe alcényle, un groupe alcynyle, un groupe aryle, un groupe hétérocyclique, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un

groupe carbamoyloxy, un groupe amino, un groupe acylamino, un groupe aminocarbonylamino, un groupe alcoxy- ou aryloxy-carbonylamino, un groupe alkyl- ou aryl-sulfonylamino, un groupe mercapto, un groupe alkyl- ou aryl-thio, un groupe thio hétérocyclique, un groupe sulfamoyle, un groupe sulfo, un groupe alkyl- ou aryl-sulfinyle, un groupe alkyl- ou aryl-sulfonyle, un groupe alkyl- ou aryl-oxycarbonyle, un groupe carbamoyle, un groupe aryl- ou hétérocyclique azo, un groupe imide, un groupe phosphino, un groupe phosphinyle, un groupe phosphinyloxy, un groupe phosphinylamino, un groupe phosphono, un groupe silyle et un groupe hydrazino ; $L^b$ et $L^c$ représentent chacun indépendamment un groupe de liaison divalent représenté par l'une quelconque des formules (L-1) à (L-12) suivantes, ou un groupe de liaison divalent consistant en 2 ou plus de 2 groupes de liaison divalents représentés chacun par l'une quelconque des formules (L-1) à (L-12) suivantes dans lesquelles les 2 ou plus de 2 groupes de liaison divalents sont liés de manière séquentielle ; et $R^b$ et $R^c$ représentent chacun indépendamment un atome d'hydrogène, un groupe alkyle substitué ou non substitué, un groupe oligooxyéthylène présentant un nombre répétitif d'un motif oxyéthylène égal à 2 ou plus, un groupe oligosiloxane présentant 2 atomes de silicium ou plus, ou un groupe trialkylsilyle substitué ou non substitué, à condition que $R^b$ et $R^c$ représentent un groupe trialkylsilyle substitué ou non substitué uniquement quand $L^b$ ou $L^c$ lié à $R^b$ ou $R^c$ est un groupe de liaison divalent représenté par la formule (L-3) suivante, et $R^b$ et $R^c$ représentent un atome d'hydrogène uniquement quand $L^b$ ou $L^c$ lié à $R^b$ ou $R^c$ est un groupe de liaison divalent représenté par l'une quelconque des formules (L-1) à (L-3) et (L-10) à (L-12) suivantes ;

Formule (2-3)

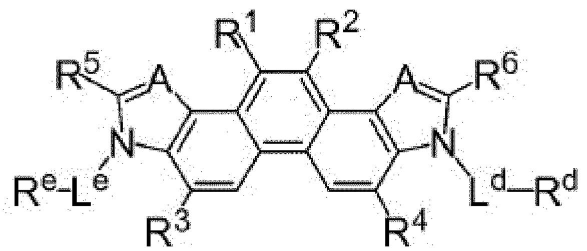

dans laquelle dans la formule (2-3), A représente $CR^8$ ou un atome d'azote ; $R^1$ à $R^6$, et $R^8$ représentent chacun indépendamment un atome d'hydrogène ou un substituant sélectionné indépendamment parmi un atome d'halogène, un groupe alkyle, un groupe alcényle, un groupe alcynyle, un groupe aryle, un groupe hétérocyclique, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un groupe carbamoyloxy, un groupe amino, un groupe acylamino, un groupe aminocarbonylamino, un groupe alcoxy- ou aryloxy-carbonylamino, un groupe alkyl- ou aryl-sulfonylamino, un groupe mercapto, un groupe alkyl- ou aryl-thio, un groupe thio hétérocyclique, un groupe sulfamoyle, un groupe sulfo, un groupe alkyl- ou aryl-sulfinyle, un groupe alkyl- ou aryl-sulfonyle, un groupe alkyl- ou aryl-oxycarbonyle, un groupe carbamoyle, un groupe aryl- ou hétérocyclique azo, un groupe imide, un groupe phosphino, un groupe phosphinyle, un groupe phosphinyloxy, un groupe phosphinylamino, un groupe phosphono, un groupe silyle et un groupe hydrazino ; $L^d$ et $L^e$ représentent chacun indépendamment un groupe de liaison divalent représenté par l'une quelconque des formules (L-1) à (L-3), (L-6), (L-7), et (L-9) à (L-12) suivantes, ou un groupe de liaison divalent consistant en 2 ou plus de 2 groupes de liaison divalents représentés chacun par l'une quelconque des formules (L-1) à (L-12) suivantes dans lesquelles les 2 ou plus de 2 groupes de liaison divalents sont liés de manière séquentielle ; et $R^d$ et $R^e$ représentent chacun indépendamment un atome d'hydrogène, un groupe alkyle substitué ou non substitué, un groupe oligooxyéthylène présentant un nombre répétitif d'un motif oxyéthylène égal à 2 ou plus, un groupe oligosiloxane présentant 2 atomes de silicium ou plus, ou un groupe trialkylsilyle substitué ou non substitué, à condition que $R^d$ et $R^e$ représentent un groupe trialkylsilyle substitué ou non substitué uniquement quand $L^d$ ou $L^e$ lié à $R^d$ ou $R^e$ est un groupe de liaison divalent représenté par la formule (L-3) suivante, et $R^d$ et $R^e$ représentent un atome d'hydrogène uniquement quand $L^d$ ou $L^e$ lié à $R^d$ ou $R^e$ est un groupe de liaison divalent représenté par l'une quelconque des formules (L-1) à (L-3) et (L-10) à (L-12) suivantes ; et

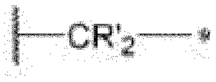

(L-1)

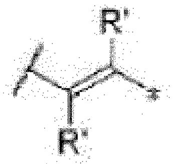

(L-2)

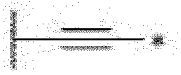

(L-3)

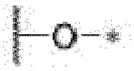

(L-4)

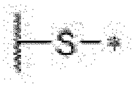

(L-5)

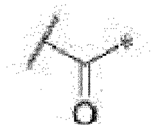

(L-6)

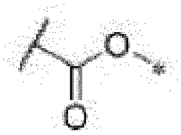

(L-7)

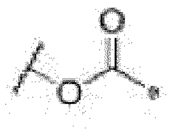

(L-8)

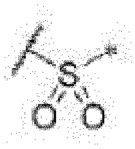

(L-9)

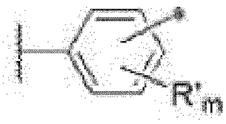

(L-10)

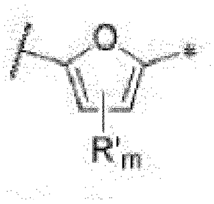

(L-11)

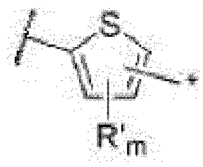

(L-12)

dans lesquelles dans les formules (L-1) à (L-12), la ligne ondulée représente une position liée au squelette phénanthrodifurane, phénanthrodithiphène ou phénanthrodipyrrole, et * représente une position liée à l'un quelconque parmi $R^a$ à $R^e$; dans la formule (L-10), m représente 4 ; dans les formules (L-11) et (L-12), m représente 2 ; dans les formules (L-1), (L-2), (L-10), (L-11) et (L-12), R' représentent chacun indépendamment un atome d'hydrogène ou un substituant; et dans les formules (L-1) et (L-2), R' peuvent former chacun indépendamment un cycle condensé avec $R^a$ à $R^e$ liés chacun à $L^a$ à $L^e$.

**3.** Composé selon la revendication 1 ou 2, dans lequel A dans la formule (1), (2-1), (2-2) ou (2-3) représente chacun indépendamment $CR^8$ à condition que $R^8$ représente un atome d'hydrogène ou un substituant sélectionné indépendamment parmi un atome d'halogène, un groupe alkyle, un groupe alcényle, un groupe alcynyle, un groupe aryle, un groupe hétérocyclique, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un groupe carbamoyloxy, un groupe amino, un groupe acylamino, un groupe aminocarbonylamino, un groupe alcoxy- ou aryloxycarbonylamino, un groupe alkyl- ou aryl-sulfonylamino, un groupe mercapto, un groupe alkyl- ou aryl-thio, un groupe thio hétérocyclique, un groupe sulfamoyle, un groupe sulfo, un groupe alkyl- ou aryl-sulfinyle, un groupe alkyl- ou aryl-sulfonyle, un groupe alkyl- ou aryl-oxycarbonyle, un groupe carbamoyle, un groupe aryl- ou hétérocyclique azo, un groupe imide, un groupe phosphino, un groupe phosphinyle, un groupe phosphinyloxy, un groupe phosphinylamino, un groupe phosphono, un groupe silyle et un groupe hydrazino.

**4.** Composé selon l'une quelconque des revendications précédentes, dans lequel A dans la formule (1), (2-1), (2-2) ou (2-3) est un groupe présentant une longueur de chaîne de liaison de 0,37 nm (3,7 Å) ou moins.

**5.** Composé selon la revendication 2, dans lequel tous parmi $L^a$ à $L^e$ dans la formule (2-1), (2-2) ou (2-3) représentent chacun un groupe de liaison divalent représenté par l'une quelconque des formules (L-1) à (L-5), (L-10), (L-11) et (L-12), ou un groupe de liaison divalent consistant en 2 ou plus de 2 groupes de liaison divalents représentés chacun par l'une quelconque des formules (L-1) à (L-5), (L-10), (L-11) et (L-12) dans lesquelles les 2 ou plus de 2 groupes de liaison divalents sont liés de manière séquentielle.

6. Composé selon la revendication 2, dans lequel tous parmi $L^a$ à $L^c$ dans la formule (2-1) ou (2-2) représentent chacun un groupe de liaison divalent représenté par l'une quelconque des formules (L-1), (L-3), (L-10) et (L-12).

7. Composé selon la revendication 2, dans lequel $L^d$ à $L^e$ dans la formule (2-3) représentent chacun indépendamment un groupe de liaison divalent représenté par l'une quelconque des formules (L-10), (L-11) et (L-12).

8. Composé selon la revendication 2, dans lequel tous parmi $R^a$ à $R^e$ dans la formule (2-1), (2-2) ou (2-3) représentent chacun un groupe alkyle substitué ou non substitué.

9. Composé selon l'une quelconque des revendications précédentes, dans lequel X représente un atome S ou un atome O.

10. Matériau semi-conducteur organique pour un dispositif semi-conducteur organique n'émettant pas de lumière, contenant le composé tel que défini dans l'une quelconque des revendications précédentes.

11. Transistor à couche mince organique, contenant le composé tel que défini dans l'une quelconque des revendications 1 à 9.

12. Solution de revêtement pour un dispositif semi-conducteur organique n'émettant pas de lumière contenant le composé tel que défini dans l'une quelconque des revendications 1 à 9.

13. Solution de revêtement selon la revendication 12, contenant en outre un liant polymère.

14. Couche mince semi-conductrice organique pour un dispositif semi-conducteur organique n'émettant pas de lumière contenant le composé tel que défini dans l'une quelconque des revendications 1 à 9.

15. Couche mince semi-conductrice organique selon la revendication 14, contenant en outre un liant polymère.

Fig. 1

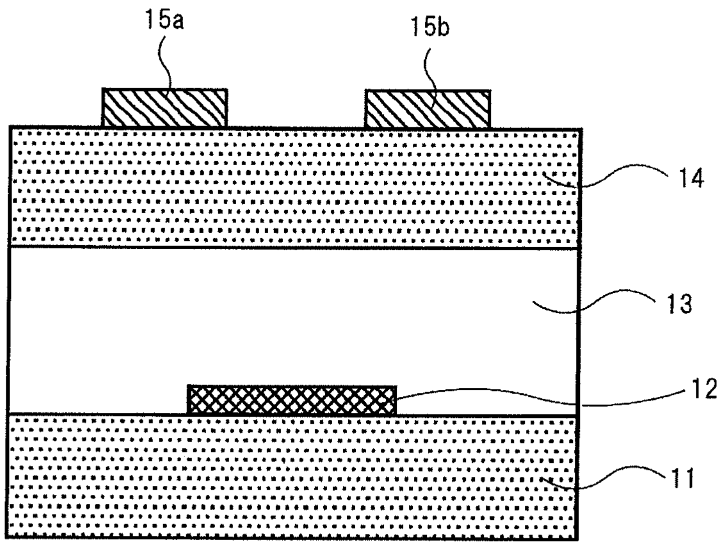


Fig. 2

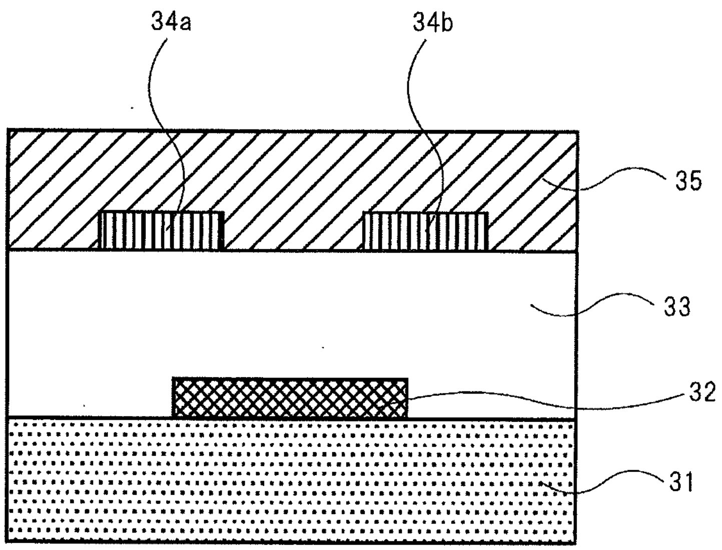

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011046687 A
- JP 2012513459 A
- WO 201146687 A
- JP 2012513459 T

### Non-patent literature cited in the description


- *Journal of Chemical Research,* 1991, vol. 3, 601-635
- *Bull. Chem. Soc. Japan,* 1991, vol. 64, 3682-3686
- *Tetrahedron Letters,* 2004, vol. 45, 2801-2803
- *Journal of Chemical Research,* 1991, vol. 3, 601-636